# EUROPEAN PATENT APPLICATION

(11) **EP 4 159 277 A1**
(43) Date of publication of application: **05.04.2023**
(21) Application number: 21812959.1
(22) Date of filing: 26.05.2021
(51) Int. Cl.: A61P 11/00, A61P 43/00, C12N 5/074, C12N 5/09, G01N 33/15, G01N 33/50, A61K 35/42, A61L 27/38

(54) **METHOD FOR PRODUCING ORGANOID FROM LUNG EPITHELIAL CELLS OR LUNG CANCER CELLS**

(30) Priority: 27.05.2020 JP 2020092303
(71) Applicant: Otsuka Pharmaceutical Co., Ltd., Tokyo 101-8535 (JP); RIKEN, Wako-shi, Saitama 351-0198 (JP)
(72) Inventor: MORIMOTO, Mitsuru, Wako-shi, Saitama 351-0198 (JP); FUJIMURA, Takashi, Osaka-shi, Osaka 540-0021 (JP)
(74) Representative: Hoffmann Eitle
(86) International application number: PCT/JP2021/019947
(87) International publication number: WO 2021/241621

(57) **Abstract**

A method of producing an organoid derived from a lung epithelial cell or a lung cancer cell, comprising culturing a sample including the lung epithelial cell or the lung cancer cell in a culture medium, wherein the culture medium contains 0-10% (v/v) extracellular matrix, and a combination of at least one selected from the group consisting of keratinocyte growth factor (KGF), fibroblast growth factor (FGF) 10, and hepatocyte growth factor (HGF); bone morphogenetic protein (BMP) inhibitor; and TGFβ inhibitor, and the culture medium is substantially free of feeder cells.

## Description

### TECHNICAL FIELD

The invention relates to a method of producing an organoid derived from a lung epithelial cell. The invention also relates to a method of producing an organoid derived from a lung cancer cell. The invention further relates to an organoid derived from a lung epithelial cell or a lung cancer cell, a regenerative medical composition comprising an organoid derived from a lung epithelial cell, and a method of screening a substance capable of treating lung cancer.

### BACKGROUND

Tissue stem cells are involved in the appropriate turnover of differentiated cells under homeostasis. Tissue stem cells play a central role in damage-induced tissue regeneration by supplying differentiated cells when tissue is injured. The lung has several kinds of tissue stem cells. Methods for culturing organoids were established for some kinds of tissue stem cells in various organ and have been investigated for practical research leading to stem cell research and regenerative medicine. Among the method for culturing organoids, a culture method using feeder cells is widely used (Patent Literature1). Methods for culturing organoids without feeder cells have been investigated in late years (Patent Literature 2,

Non-Patent Literature 1).

### CITATION LIST

Patent Literature 1: JP-A 2016-513469
Patent Literature 2: JP-A 2018-503360

Non-Patent Literature 1: Biochemical and Biophysical Research Communications, Volume 515, Issue 4, 6 August 2019, Pages 579-585

### SUMMARY

The present disclosure mainly provides a new method of producing an organoid derived from a lung epithelial cell or a lung cancer cell.

### SOLUTION TO PROBLEM

The present disclosure provides a method of producing an organoid derived from a lung epithelial cell or a lung cancer cell, the method comprising culturing a sample including the lung epithelial cell or the lung cancer cell in a culture medium, wherein the culture medium contains 0-10% (v/v) extracellular matrix, and a combination of at least one selected from the group consisting of keratinocyte growth factor (KGF), fibroblast growth factor (FGF) 10, and hepatocyte growth factor (HGF); bone morphogenetic protein (BMP) inhibitor; and TGFβ inhibitor, and the culture medium is substantially free of feeder cells.

The present disclosure further provides an organoid derived from a lung epithelial cell or a lung cancer cell, the organoid being produced by the above-described method. The present disclosure provides an organoid derived from a lung epithelial cell, the organoid comprising an intracavity and a cell layer facing the intracavity.

The present disclosure further provides a regenerative medical composition comprising an organoid derived from a lung epithelial cell produced by the above-described method.

The present disclosure further provides a method of screening a substance capable of treating lung cancer, the method comprising contacting a subject substance with an organoid derived from a lung cancer cell, produced by the above-described method, measuring the organoid after contact with the subject substance, and comparing the measured value from the organoid after contact with the subject substance to a control value.

The present disclosure further provides a method of producing an organoid derived from a lung epithelial cell or a lung cancer cell, the method comprising culturing a sample including the lung epithelial cell or the lung cancer cell in a culture medium, wherein the culture medium contains 0-10% (v/v) extracellular matrix, and the culture medium is substantially free of feeder cells; and the following combination: a combination including an agent capable of activating Wnt signaling, bone morphogenetic protein(BMP) inhibitor, TGFβ inhibitor, and hepatocyte growth factor(HGF); a combination including either or both of keratinocyte growth factor(KGF) and fibroblast growth factor(FGF) 10, BMP inhibitor, TGFβ inhibitor, and HGF; or a combination including either or both of KGF and FGF10, an agent capable of activating Wnt signaling, BMP inhibitor, TGFβ inhibitor, and HGF.

The present disclosure provides the following inventions:
[item 1] A method of producing an organoid derived from a lung epithelial cell or a lung cancer cell, comprising culturing a sample including the lung epithelial cell or the lung cancer cell in a culture medium, wherein the culture medium contains 0-10% (v/v) extracellular matrix, and a combination of at least one selected from the group consisting of keratinocyte growth factor (KGF), fibroblast growth factor (FGF) 10, and hepatocyte growth factor (HGF); bone morphogenetic protein (BMP) inhibitor; and TGFβ inhibitor, and the culture medium is substantially free of feeder cells.
[Item 2] The method according to Item 1, wherein the combination further comprises either or both of an agent capable of activating Wnt signaling and Rho-kinase (ROCK) inhibitor.
[Item 3] The method according to Item 2, wherein the combination is a combination of the KGF, the BMP inhibitor, and the TGFβ inhibitor; a combination of the KGF, the agent capable of activating Wnt signaling, the BMP inhibitor, and the TGFβ inhibitor; a combination of the FGF10, the BMP inhibitor, and the TGFβ inhibitor; a combination of the FGF10, the agent capable of activating Wnt signaling, the BMP inhibitor, and the TGFβ inhibitor; a combination of the HGF, the BMP inhibitor, and the TGFβ inhibitor; a combination of the HGF, the agent capable of activating Wnt signaling, the BMP inhibitor, and the TGFβ inhibitor; a combination of the KGF, the FGF10, the HGF, the BMP inhibitor, and the TGFβ inhibitor; a combination of the KGF, the FGF10, the HGF, the agent capable of activating Wnt signaling, the BMP inhibitor, and the TGFβ inhibitor; a combination of the ROCK inhibitor, the KGF, the FGF10, the HGF, the BMP inhibitor, and the TGFβ inhibitor; or a combination of the ROCK inhibitor, the KGF, the FGF10, the HGF, the agent capable of activating Wnt signaling, the BMP inhibitor, and the TGFβ inhibitor.
[Item 4] The method according to any one of Items 1-3, wherein the BMP inhibitor is Noggin and/or the TGFβ inhibitor is SB431542.
[Item 5] The method according to any one of Items 2-4, wherein the agent capable of activating Wnt signaling is CHIR99021 and/or the ROCK inhibitor is Y-27632.
[Item 6] The method according to any one of Items 1-5, wherein lung epithelial stem cells included in the sample comprise at least one cell type selected from the group consisting of basal cell, club cell, bronchioalveolar stem cell, and alveolar epicelial cell.
[Item 7] The method according to any one of Items 1-5, wherein the lung epithelial cell included in the sample essentially consists of alveolar epicelial type 2 cell (hereinafter "AT2 cell"); essentially consist of club cell, or essentially consist of a combination of basal cell and club cell.
[Item 8] The method according to any one of Items 1-5, wherein lung epithelial cell included in the sample essentially consist of alveolar epicelial type 2 cell, or essentially consists of club cell.
[Item 9] The method according to any one of items 1-8, comprising selecting club-I cell, AT2 cell, or club-II cell from the sample, based on at least one of cell markers corresponding to respective club-I cell, AT2 cell, and club-II cell listed in Table A and homologs thereof, before culturing the sample in the culture medium.

| Table A | | |
|---|---|---|
| club-I cell | AT2 cell | club-II cell |
| Cbr2 | Lamp3 | Tff2 |
| Hp | Lyz2 | Reg3q |
| Cyp2f2 | Napsa | Bpifb1 |
| Prdx6 | Slc34a2 | Muc5b |
| Scqb1a1 | Lyz1 | Sult1d1 |
| Ldhb | Rnase4 | Fxyd3 |
| Ces1d | Hc | Scgb3a1 |
| Cckar | Cd74 | Lypd2 |
| Selenbp1 | Scd1 | Chad |
| Gstm2 | Sftpc | S100a6 |
| Scnn1b | Lgi3 | Pglyrp1 |
| Scgb1c1 | Cldn18 | Aldh3a1 |
| Plpp3 | Etv5 | Bpifa1 |
| Ephx1 | Cxcl15 | Gsto1 |
| Dcxr | S100g | Lgals3 |
| Alas1 | Elovl1 | Pigr |
| Retnla | Fas | Scgb3a2 |
| Sec1413 | H2-Aa | Ltf |
| Ptqr1 | Fabp5 | Qsox1 |
| Krtap17-1 | Rn4.5s | Cvp2a5 |
| | | Cpd |
| | | Ly6a |
| | | Perp |
| | | Kcne3 |
| | | Il13ra1 |
| | | Slc15a2 |
| | | Cd14 |

[item 10] The method according to any one of Items 1-9, further comprising culturing, in a culture medium free of the above-described combination, the cells cultured in the culture medium containing the above-described combination.
[Item 11] An organoid derived from a lung epithelial cell or a lung cancer cell, produced by a method according to any one of Items 1-10.
[Item 12] An organoid derived from a lung epithelial cell, comprising an intracavity and a cell layer facing the intracavity, wherein the lung epithelial cell is alveolar cell, and the cell layer essentially consists of: AT2 cells; a combination of AT2 cells with cells expressing an AT2 cell marker and a bronchial epithelium marker; or cells expressing a bronchial epithelium marker, or the lung epithelial cell is airway cell, and the cell layer essentially consists of a combination of basal cells with cells expressing a basal cell marker and a bronchial epithelium marker.
[Item 13] The organoid according to Item 12, wherein the lung epithelial cell is alveolar cell, the cell layer essentially consists of AT2 cells, the AT2 cells express either or both of HT2-280 and SFTPC, and the HT2-280 locally localizes in or near cell membranes of the AT2 cells, the cell membranes facing the intracavity.
[Item 14] The organoid according to Item 12, wherein the AT2 cell marker is either or both of HT2-280 and SFTPC, the bronchial epithelium marker is SOX2, and/or the basal cells marker is KRT5.
[Item 15] A regenerative medical composition comprising an organoid derived from a lung epithelial cell, produced by a method according to any one of Items 1-10, an organoid derived from a lung epithelial cell according to any one of Items 12-14, and/or cells from the organoid.
[Item 16] A regenerative medical composition comprising lung epithelial cells, the lung epithelial cells essentially consisting of club-I cell, AT2 cell, or club-II cell expressing at least one of cell markers listed in Table A and homologs thereof.

**Table A**

| club-I cell | AT2 cell | club-II cell |
|---|---|---|
| Cbr2 | Lamp3 | Tff2 |
| Hp | Lyz2 | Reg3g |
| Cyp2f2 | Napsa | Bpifb1 |
| Prdx6 | Slc34a2 | Muc5b |
| Scgb1a1 | Lyz1 | Sult1d1 |
| Ldhb | Rnase4 | Fxyd3 |
| Ces1d | He | Scgb3a1 |
| Cckar | Cd74 | Lypd2 |
| Selenbp1 | Scd1 | Chad |
| Gstm2 | Sftpc | S100a6 |
| Scnn1 b | Lgi3 | Pglyrp1 |
| Scgb1c1 | Cldn18 | Aldh3a1 |
| Plpp3 | Etv5 | Bpifa1 |
| Ephx1 | Cxcl15 | Gsto1 |
| Dcxr | S100g | Lgals3 |
| Alas1 | Elovl1 | Pigr |
| Retnla | Fas | Scgb3a2 |
| Sec14l3 | H2-Aa | Ltf |
| Ptgr1 | Fabp5 | Qsox1 |
| Krtap17-1 | Rn4.5s | Cyp2a5 |
| | | Cpd |
| | | Ly6a |
| | | Perp |
| | | Kcne3 |
| | | Il13ra1 |
| | | SIc15a2 |
| | | Cd14 |

[Item 17] A method of screening a substance capable of treating lung cancer, comprising contacting a subject substance with an organoid derived from a lung cancer cell, produced by a method according to any one of Items 1-10, measuring the organoid after contact with the subject substance, and comparing the measured value from the organoid after contact with the subject substance to a control value.
[Item 18] A pharmaceutical composition comprising an agent for treating lung injury or lung disease and lung epithelial cells essentially consisting of club-II cells, the club-II cell being selected from a sample including lung epithelial cells based on at least one of cell markers corresponding to club-II cell listed in Table A and homologs thereof.

### BRIEF DESCRIPTION OF DRAWINGS

FIG. 1 is a scatter plot of lung epithelial stem cells reflecting from fluorescence intensities of green fluorescent protein (GFP) emitted from the lung epithelial stem cells obtained from an SFTPC-GFP mouse.
FIG. 2 is a bar graph showing the formation rates of organoids derived from lung epithelial stem cells in a GFP^{hi} fraction, the organoids being formed in a culture medium containing an extracellular matrix as a scaffold.
FIG. 3 is a bar graph showing the major axis of organoids derived from lung epithelial stem cells in a GFP^{hi} fraction, the organoids being formed in a culture medium containing an extracellular matrix as a scaffold.
FIG. 4 is a bar graph showing the formation rates of organoids derived from lung epithelial stem cells in a GFP^{lo} fraction, the organoids being formed in a culture medium containing an extracellular matrix as a scaffold.
FIG. 5 is a bar graph showing the major axis of organoids derived from lung epithelial stem cells in a GFP^{lo} fraction, the organoids being formed in a culture medium containing an extracellular matrix as a scaffold.
FIG. 6 is a bar graph showing the formation rates of organoids derived from lung epithelial stem cells, the organoids being formed in a culture medium containing extracellular matrix as a dispersed component.
FIG. 7 is a bar graph showing the formation percentages of organoids derived from lung cancer cells.
FIG. 8 is a scatterplot of lung epithelial stem cells reflecting from fluorescence intensities of GFP emitted from the lung epithelial stem cells obtained from an SFTPC-GFP mouse.
FIG. 9 is a bar graph showing the expression levels of each marker gene for individual GFP fractions.
FIG. 10 shows fluorescent images of organoids derived from lung epithelial stem cells in the GFP^{neg} fraction.
FIG. 11 shows fluorescent images of organoids derived from lung epithelial stem cells in the GFP^{lo} fraction.
FIG. 12 fluorescent images of organoids derived from lung epithelial stem cells in the GFP^{hi} fraction.
FIG. 13A is a bar graph showing the formation rates of organoids derived from lung epithelial stem cells in the GFP^{hi} fraction, the organoids being formed in a culture medium containing an extracellular matrix as a scaffold. FIG. 13B is a graph showing the major axis of the organoids derived from the lung epithelial stem cells in the GFP^{hi} fraction, the organoids being formed in the culture medium containing the extracellular matrix as a scaffold.
FIG. 14A is a bar graph showing the formation rates of organoids derived from lung epithelial stem cells in the GFP^{lo} fraction, the organoids being formed in a culture medium containing an extracellular matrix as a scaffold. FIG. 14B is a graph showing the major axis of the organoids derived from the lung epithelial stem cells in the GFP^{lo} fraction, the organoids being formed in the culture medium containing the extracellular matrix as a scaffold.
FIG. 15A is a bar graph showing the formation rates of organoids derived from lung epithelial stem cells in the GFP^{neg} fraction, the organoids being formed in a culture medium containing an extracellular matrix as a scaffold. FIG. 15B is a graph showing the major axis of the organoids derived from the lung epithelial stem cells in the GFP^{neg} fraction, the organoids being formed in the culture medium containing the extracellular matrix as a scaffold.
FIG. 16A is a schematic showing the donor mouse and the recipient mouse in Example 3.
FIG. 16B is a diagram showing the scheme of Example 3. FIG. 16C is a fluorescent image showing a frozen section of the lung of mouse into which cells derived from organoids were injected. FIG. 16D shows enlarged fluorescent images of the area surrounded by the white square in FIG. 16C.
FIG. 17A is a microscopic image showing organoids formed from human alveolar cells.
FIG. 17B is a microscopic image showing organoids formed from human airway cells.
FIG. 18A is a fluorescent image of a pulmonary alveolar-type organoid formed from human alveolar cells. FIG. 18B is a fluorescent image of a bronchioalveolartype organoid formed from human alveolar cells. FIG. 18C is a fluorescent image of a bronchial-type organoid formed from human alveolar cells.

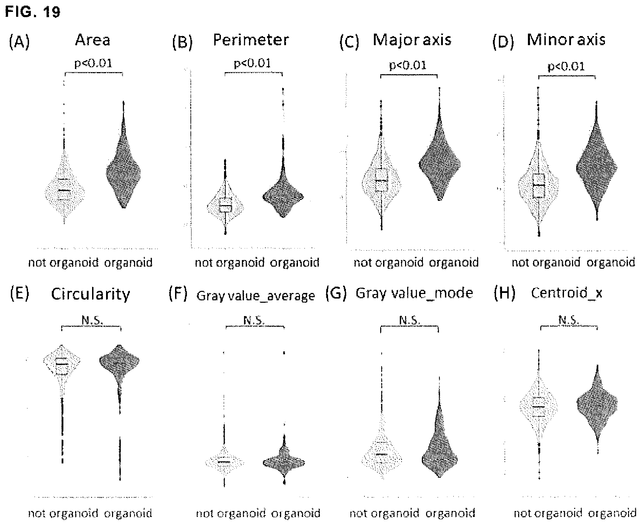

FIG. 19A is a graph of the areas of cells constituting organoids cultured from a cell population. FIG. 19B is a graph of the perimeters of the cells constituting the organoids cultured from the cell population. FIG. 19C is a graph of the major axis of the cells constituting the organoids cultured in the cell population. FIG. 19D is a graph of the minor axis of the cells constituting the organoids cultured from the cell population. FIG. 19E is a graph of the circularities of the cells constituting the organoids cultured from the cell population. FIG. 19F is a graph of the gray value (averages) of the cells constituting the organoids cultured from the cell population. FIG. 19G is a graph of the gray value (modes) of the cells constituting the organoids cultured from the cell population. FIG. 19H is a graph of the centroids of the cells constituting the organoids cultured from the cell population.
FIG. 20A is a graph of the areas of cells constituting organoids cultured from single cells.
FIG. 20B is a graph of the perimeters of the cells constituting the organoids cultured from the single cells. FIG. 20C is a graph of the major axis of the cells constituting the organoids cultured in the single cells. FIG. 20D is a graph of the minor axis of the cells constituting the organoids cultured from the single cells. FIG. 20E is a graph of the circularities of the cells constituting the organoids cultured from the single cells. FIG. 20F is a graph of the gray value (averages) of the cells constituting the organoids cultured from the single cells.
FIG. 20G is a graph of the gray value (modes) of the cells constituting the organoids cultured from the single cells. FIG. 20H is a graph of the centroids of the cells constituting the organoids cultured from the single cells.
FIG. 21A is a scatterplot of the expression levels of Scgb1a1 in individual clusters. FIG. 21B is a scatterplot of the expression levels of Sftpc in the individual clusters. FIG. 21C is a scatterplot of the cell types in the individual clusters. FIG. 21D is a graph of the areas of the individual clusters.
FIG. 22 is a graph showing nine cell surface markers highly expressed in the cells corresponding to Cluster 2.
FIG. 23A is a bar graph of the expression levels of marker genes in CD14^{+/-} cells. FIG. 23B is a graph of the areas of the CD14^{+/-} cells.
FIG. 24A shows microscopic images of CD14^{+/-} cells on day 9 after culturing. FIG. 24B is a bar graph of the percentages of organoid formation derived from the CD14^{+/-} cells.
FIG. 25 is a bar graph of the expression levels of marker genes in organoids derived from CD14^{+/-} cells.
FIG. 26A shows fluorescent images of an immunostained organoid derived from CD14⁺ cells on day 9 after culturing. FIG. 26B shows fluorescent images of an immunostained organoid derived from CD14⁺ cells on day 12 after culturing.
FIG. 27A is a bar graph of the number of clusters observed in the lungs into which CD14^{+/-} cells were injected. FIG. 27B is a graph of the number of cells constituting the clusters observed in the lungs into which CD14^{+/-} cells were injected.
FIG. 28 shows fluorescent images indicating that CD14⁺ cells injected into bleomycin-injured lungs differentiated into alveolar epithelial type 1 cells (hereafter "AT1 cells") or alveolar epithelial type 2 cells (hereinafter "AT2 cells").
FIG. 29 shows fluorescent images indicating that CD14⁺ cells injected into bleomycin-injured lungs differentiated into Ciliated cells.

### DESCRIPTION OF EMBODIMENTS

### [Method of producing an organoid derived from a lung epithelial cell or a lung cancer cell]

An aspect of the present disclosure is to provide a method of producing an organoid derived from a lung epithelial cell or a lung cancer cell. The method comprises culturing a sample including the lung epithelial cell or the lung cancer cell in a culture medium, wherein the culture medium contains 0-10% (v/v) extracellular matrix and addition agents according to the present disclosure, and the culture medium is substantially free of feeder cells.

The term "organoid" herein refers to a living tissue-like cellular aggregate produced *in vitro* in three dimensions. In the context of the organoid, "like living tissue" or "living tissue-like" means that an organoid has an anatomical structure similar to living tissue. Most cells constituting the organoid possess, for example, the capability to differentiate and proliferate. Cultured cells constituting the organoid may be differentiated into various types of cells according to known methods. For example, the cultured cells constituting the organoid may be differentiated into various types of cells by adding or excluding addition agents from the culture medium. For example, the cultured cells constituting the organoid may be induced to differentiate by culturing them in a culture medium free of addition agents of the present disclosure. The organoid may contain, in part, differentiated cells or cells possessing no capacity to differentiate. For example, the organoid may be produced according to known methods for culturing cells. For example, the organoid may be produced by maintaining a given cell in a culture medium containing addition agents of the disclosure in an apparatus for culturing cells. In an embodiment, the organoid is an organoid derived from a lung epithelial stem cell or a lung cancer cell.

In an embodiment, the organoid is an aggregate of cultured cells, the aggregate being at least 50 µm in diameter when observing it with a microscope. When the cultured cells aggregate is elliptical, the diameter is the length of a long axis. The diameter of the cultured cells aggregate being complex in shape is the diameter of the circumscribed circle of the cells aggregate. The efficiency of organoid formation, Colony Forming Efficiency (CFE), is a percentage [%] calculated by dividing the number of organoids whose diameter is not less than 50 µm in a cultured product on day 6 after culturing a sample including lung epithelial cells or lung cancer cells in a culture medium by the number of the lung epithelial cells or lung cancer cells added to the culture medium.

The term "stem cell" herein refers to a cell capable of proliferating itself (referred to as "proliferative capacity") and differentiating into a specific cell type (referred to as "differentiation capacity"). For example, stem cells can differentiate into epithelial stem cells. For example, stem cells can also proliferate while maintaining their differentiated capacity according to known methods. For example, stem cells can also proliferate while differentiating into a specific cell type according to known methods.

The term "lung epithelial cell" herein refers to a cell that constitutes epithelial tissue of the lung. The lung epithelial cell may be, for example, a Club cell, Ciliated cell, neuroendocrine cell, Basal cell, goblet cell, alveolar epithelial cell, or lung epithelial stem cell capable of differentiating into the above cells. Alveolar epithelial cells may be, for example, either or both of alveolar epithelial type 1 cell (Type I alveolar epithelial cell: AT1 cell) and alveolar epithelial type 2 cell (AT2 cell). The term "lung epithelial stem cell" herein refers to a cell that is present in lung tissue and is capable of differentiating into specific lung epithelial cells and proliferating. The lung tissue includes, for example, trachea, bronchus, bronchiole, and pulmonary alveoli.

The lung epithelial stem cell possesses the capability to differentiate into at least one cell type, for example, selected from the group consisting of basal cell, club cell, and alveolar epithelial type 2 cell. The lung epithelial stem cell may be, for example, bronchioalveolar stem cell (Bronchioalveolar stem cells: BASCs). The lung epithelial stem cell may be prepared according to known methods (e.g., methods described in Patent Literature2).

Basal cells possess the capability to differentiate into club cells and ciliated cells during the regeneration of injured trachea under homeostatic regulation. Club cells possess the capability to differentiate into ciliated cells during the regeneration of injured trachea under homeostatic regulation. Club cells possess the capability to differentiate into AT2 cells and AT1 cells during the regeneration of severely injured pulmonary alveoli.

The term "alveolar cell" herein refers to a cell obtained from pulmonary alveoli tissue. The alveolar cell may be prepared from the pulmonary alveoli tissue according to known methods. Alveolar cells are commercially available. Alveolar cells may include alveolar epicelial cells. In one example, the alveolar cells include either or both of AT1 cells and AT2 cells. The term "airway cell" herein refers to a cell obtained from airway tissue. The airway cell may be prepared from airway tissue according to known methods. Airway cells are commercially available.

The term "sample including lung epithelial cell" herein may be prepared, for example, from mammalian lung tissue. For example, a sample including a lung epithelial cell may be prepared from mammalian lung tissue using a predetermined cell marker (e.g., a specific protein on the cell membrane surface) as an indicator. The predetermined cell marker may be, for example, EPCAM. A sample including lung epithelial cells may be used to prepare a sample including the desired type of lung epithelial cell. For example, when preparing a sample including the desired type of lung epithelial cells, a nerve growth factor receptor (NGFR) may be used as a cell marker for basal cells (Proc. Natl. Acad. Sci. USA, vol.106 (31): 12771-12775). For example, the AT2-280 may be used as a cell marker for AT2 cells (Journal of Histochemistry & Cytochemistry, vol. 58 (10): 891-901, 2010). For example, the cell markers may be specific target RNA in living cells. The target RNA may be EPCAM, Sftpc, KRT-5, or Scgb1a1.

When a particular protein on the cell membrane surface is used as a predetermined cell marker, fluorophore-labeled antibodies against the protein may be used, and the fluorescence emitted from the fluorophore may be used as an indicator. Known devices (e.g., FACS) may be used to prepare cells with a cell marker as an indicator. When a target RNA is used as a predetermined cell marker, it may comprise fractionating a sample including lung epithelial cells with FACS in predetermined conditions and identifying a cell type in the obtained sample fractionated above based on the target RNA (e.g., RT-PCR).

For example, samples including lung epithelial cells are prepared by cutting mammalian lung tissue into small pieces, subjecting the lung tissue pieces to the treatment with protease (e.g., collagenase, dispase, elastase, or trypsin), and then transferring and suspending them in a predetermined solution (e.g., basal medium). The prepared cell suspension may be filtered and/or centrifuged to remove foreign material, such as tissue debris. In an embodiment, the sample including lung epithelial cells may be prepared from the lung tissue of a mammalian adult, immature, newborn infant, or infant. In an embodiment, the sample including the lung epithelial cells may include lung epithelial cells genetically modified (but not including modifications to confer pluripotency) to possess the desired characteristics after preparation from mammalian lung tissue

In an embodiment, lung epithelial cells included in the sample comprise at least one cell type selected from the group consisting of basal cell, club cell, bronchioalveolar stem cell, and alveolar epithelial cell (e.g., either or both AT1 cell and AT2 cell). Samples including lung epithelial cells are commercially available. The sample including lung epithelial cells may be, for example, a sample including human alveolar cells or a sample including human airway cells. In an embodiment, the lung epithelial cells included in the sample may consist essentially of at least one, at least two, or at least three cell types selected from the group consisting of basal cell, club cell, bronchuslung epithelial stem cell, and alveolar epicelical cell. In an embodiment, the lung epithelial cells included in the sample consist essentially of alveolar epithelial type 2 cell. In an embodiment, the lung epithelial cells included in the sample consist essentially of club cells. In an embodiment, the lung epithelial cells included in the sample consist essentially of a combination of basal cells and club cells. The phrase "consist essentially of" in the context of a lung epithelial cell does not exclude "consist only of." In an embodiment, the lung epithelial cells included in the sample consist only of a specific type of cells. The lung epithelial cells included in the sample comprise, for example, a specific type of cells of not less than 80%, not less than 85%, not less than 90%, not less than 95%, not less than 97%, not less than 98%, or not less than 99%.

In an embodiment, a method according to the aspect further comprises preparing a sample including a lung epithelial cell or a lung cancer cell from lung tissue of a mammal. The sample preparation may be performed according to the methods disclosed herein or known methods. The term "lung cancer cell" herein is, for example, a tumor cell derived from a lung tumor. The lung cancer cell may be, for example, a tumor cell that circulates or does not circulate in the blood. Lung cancer cells may be, for example, commercially available or prepared from lung tissue that includes tumor cells according to known methods. The lung cancer cell is, for example, a lung adenocarcinoma cell.

The term "sample including lung cancer cell" herein may be, for example, fractionated from lung tissue of a mammal suffering from lung cancer by using a lung cancer cell marker as an indicator. The lung cancer cell marker may be, for example,SOX2, CD24, CD44, CD133, CD166, and ESA, or a combination thereof. For example, the lung cancer cell may be fractionated with FACS, in which a lung cancer cell marker is used as an indicator. For example, a sample that includes lung cancer cells may be prepared by suspending commercially available lung cancer cells cultured and proliferated in a predetermined solution. For example, samples containing lung cancer cells may be prepared according to the methods described for the samples including lung epithelial cells. A sample including lung cancer cells may also include, for example, normal lung epithelial cells.

The term "mammal" herein is, for example, a human or non-human mammal. The non-human mammal may be, for example, rodent such as mouse, rat, guinea pig, or hamster; non-human primate such as chimpanzee; even-toed ungulate such as cow, goat, or sheep; odd-toed ungulate such as horse, and companion animal such as rabbit, dog, or cat. in an embodiment, the mammal is rodent or non-human primate. In an embodiment, the mammal is human.

The term "organoid derived from lung epithelial cell" or "organoid derived from lung cancer cell" herein refers to a biological tissue-like construct, *in vitro* produced in three dimensions, the construct being derived from lung epithelial cell(s) or lung cancer cell(s). In an embodiment, the organoid derived from lung epithelial cells or lung cancer cells is an organoid formed by culturing the lung epithelial cells or lung cancer cells in a culture medium containing addition agents of the disclosure. In an embodiment, the organoid derived from the lung epithelial cells includes an intracavity and a cell layer facing the intracavity.

The term "culture medium" herein refers to a liquid containing components required for culturing cells. The culture medium includes, for example, a basal medium and addition agents of the disclosure. The culture medium may be, for example, prepared by combining the basal medium with the addition agents of the disclosure. For example, the culture medium may be prepared by mixing a mixture that includes predetermined amounts of each component (in solid) for the basal medium and predetermined amounts of the addition agents (in solid) of the disclosure with pure water so as to make them predetermined concentrations. The culture medium may further contain either or both of feeder cells that assist stem cells in proliferating and extracellular matrix. For example, the culture medium may be prepared by adding to a mixture of the basal medium and the addition agents of the disclosure, either or both of feeder cells that assist stem cells in proliferating and extracellular matrix.

The term "basal medium" herein may be a known medium for culturing cells and be, for example, Dulbecco's Modified Eagle's Medium (DMEM), Minimum Essential Medium (MEM), Basal Medium Eagle × (BME) medium, or DMEMIF12. In an embodiment, the basal medium is DMEM/F12.

The term "addition agents" herein refers to a combination of two or more substances that affect the efficiency of organoid formation derived from lung epithelial cell(s) or lung cancer cell(s). The addition agents may be, for example, a combination of two or more substances that improve the efficiency of organoid formation derived from lung epithelial cells or lung cancer cells. The addition agents may include, for example, a substance such that cells constituting a formed organoid can maintain the differentiated capacity or induce the differentiation into a specific cell type.

In an embodiment, the additional agents may be a combination comprising or consisting of at least one selected from the group consisting of keratinocyte growth factor (KGF), hepatocyte growth factor (HGF), and fibroblast growth factor (FGF) 10; bone morphogenetic protein (BMP) inhibitor (e.g., Noggin); and TGFβ inhibitor (e.g., SB431542). Preferably, the combination further comprises an agent capable of activating Wnt signaling (e.g., CHIR99021) in view of the efficient formation of AT2 organoids. The combination may further comprise Rock inhibitor (e.g., Y-27632).

In view of the efficient organoid formation, the additional agents may be a combination comprising or consisting of an agent capable of activating Wnt signaling (e.g., CHIR99021), BMP inhibitor (e.g., Noggin), TGFβ inhibitor (e.g., SB431542), and HGF. The combination may further comprise Rock inhibitor.

In view of more efficient organoid formation, the addition agents may be a combination comprising or consisting of either or both of KGF and FGF10; BMP inhibitor (e.g., Noggin); TGFβ inhibitor (e.g., SB431542); and HGF. In an embodiment, the additional agents are a combination comprising or consisting of KGF, FGF10, BMP inhibitor (e.g., Noggin), TGFβ inhibitor (e.g., SB431542), and HGF. In view of the efficient formation of AT2 organoids, the combination preferably further comprises an agent capable of activating Wnt signaling (e.g., CHIR99021). The combination may further comprise Rock inhibitor.

In view of further efficient organoid formation, the addition agents are a combination comprising or consisting of KGF, FGF10, an agent capable of activating Wnt signaling (e.g., CHIR99021), BMP inhibitor (e.g., Noggin), TGFβ inhibitor (e.g., SB431542), HGF, and FGF10. The additional agents are, for example, a combination comprising or consisting of KGF, FGF10, an agent capable of activating Wnt signaling (e.g., CHIR99021), BMP inhibitor (e.g., Noggin), TGFβ inhibitor (e.g., SB431542), HGF, FGF10, and Rock inhibitor.

The term "keratinocyte growth factor (KGF)" herein refers to a substance that stimulates epithelial cells, constituting the skin surface and the mucosa of the oral cavity, stomach, and intestinal tract, to proliferate. For example, KGFs are commercially available. For example, the KGF is 10-250 ng/mL, 20-100 ng/mL, and 30-70 ng/mL, preferably 50ng/mL in concentration when included in a culture medium. The KGF is occasionally abbreviated herein as "K."

The term "hepatocyte growth factor (HGF)" herein refers to a cytokine known as the most potent growth factor of hepatocytes. The HGF generally has a heterodimeric structure with a disulfide bond between a heavy chain of about 60,000 in molecular weight and a light chain of about 35,000 in molecular weight. For example, HGFs are commercially available. For example, the HGF is 5-150 ng/mL, 10-75 ng/mL, and 20-40 ng/mL, preferably 30 ng/mL in concentration when included in a culture medium. The HGF is occasionally abbreviated herein as "H."

The term "fibroblast growth factor (FGF)10" herein refers to a signaling factor that stimulates epithelial cells to proliferate, migrate, and differentiate, promoting damaged skin or mucosal tissue repair. For example, FGF10s are commercially available. For example, the FGF10 is 10-250 ng/mL, 20-100 ng/mL, and 30-70 ng/mL, preferably 50 ng/mL in concentration when included in a culture medium. The FGF10 is occasionally abbreviated herein as "F10".

The term "agent capable of activating Wnt signaling" herein refers to a substance that activates transcription via T-cell transcription factor (TCF/LEF) in cells. The agent capable of activating Wnt signaling is also known as Wnt agonist. The agent capable of activating Wnt signaling may be, for example, a substance that inhibits serine-threonine protein kinase GSK-3, which mediates the phosphorylation on amino acid residues of serine and threonine (referred to as "GSK inhibitor"), Wnt family protein, or inhibitor of β-catenin degradation, or a combination of two or more thereof. For example, the agents capable of activating Wnt signaling are commercially available.

An agent capable of activating Wnt signaling may be, for example, GSK-3 inhibitor. The GSK-3 inhibitor may be, for example, SB216763 (Chemscene LLC), CHIR-98014 (Abeam), TWS119 (Cayman Chemical), or CHIR99021 (Sigma), or a combination of two or more thereof. The agent capable of activating Wnt signaling may be, for example, the Wnt family protein (referred to as "Wnt ligand"). The Wnt family protein may be, for example, any one of 19 Wnt genes-derived proteins known in the field or a combination of two or more thereof. For example, the Wnt family protein may be Wnt-1, -2, -3a, -6, -7a, -7b, -8a, -9a, -10a, or -16, or a combination of two or more thereof. The Wnt family protein may be, for example, Wnt1. The agent capable of activating Wnt signaling may be, for example, β-catenin degradation inhibitor. The β-catenin degradation inhibitor may be, for example, a substance that inhibits the phosphorylation of β-catenin (e.g., UBE1-41).

In an embodiment, the agent capable of activating Wnt signaling is GSK-3 inhibitor. The GSK-3 inhibitor is preferably CHIR99021. For example, the agent capable of activating Wnt signaling is an amount such that it can exert the inhibition effect corresponding to CHIR99021 of 0.5-15 µM, 1-7 µM, or 2-5 µM, preferably 3 µM when included in a culture medium. CHIR99021 is occasionally abbreviated herein as "C."

The term "BMP inhibitor" herein refers to a substance that binds to a BMP molecule to form a complex. The BMP inhibitor may be, for example, small molecule compound, protein (e.g., antibodies), DNA, RNA, small interfering RNA, or antisense oligonucleotide. For example, BMP inhibitors are commercially available. The BMP inhibitor may be, for example, Noggin (Bmp inhibitor), Chordin and chordin-like proteins (R&D systems) comprising chordin domains, Follistatin and follistatin-related proteins (R&D systems) comprising a follistatin domain, DAN and DAN-like proteins (R&D systems) comprising a DAN cysteine-knot domain, sclerostinISOST (R&D systems), decorin (R&D systems), or alpha-2 macroglobulin (R&D systems), or a combination of two or more thereof. In an embodiment, the BMP inhibitor is Noggin. For example, the BMP is an amount such that it can exert the inhibition effect corresponding to Noggin of 20-500 ng/ml, 40-250 ng/ml, and 80-125 ng/ml, preferably 100ng/ml when included in a culture medium. Noggin is occasionally abbreviated herein as "N."

The term "TGFβ inhibitor" herein refers to a substance that inhibits TGFβ signaling. The TGFβ inhibitor is, for example, a substance that inhibits intracellular signaling through the phosphorylation of Smad2/3 protein by Type 1 receptor (ALK5) serinelthreonine kinase activated by binding of TGFβ. The TGF-β inhibitor may be, for example, small molecule compound, protein (e.g., antibody), DNA, RNA, small interfering RNA, or antisense oligonucleotide. The TGF-β inhibitors may be A83-01 (Abcam), SB-431542 (Calbiochem), SB-505124 (R&D systems), SB-525334 (Chemscene LLC), LY364947 (Sigma-Aldrich), SD-208 (Sigma-Aldrich), or SJN2511 (R&D systems), or a combination of two or more thereof. In an embodiment, the TGFβ inhibitor is SB431542. For example, the TGFβ inhibitor is an amount such that it can exert the inhibition effect corresponding to SB431542 of 2-50 µM, 4-25 µM, or 8-12 µM, preferably 10 µM when included in a culture medium. SB431542 is occasionally abbreviated herein as "S."

The term "epidermal growth factor (EGF)" herein refers to a protein that regulates cell growth and proliferation by binding to epidermal growth factor receptor (EGFR) on the cell surface. In general, EGF is about 6,000 daltons in molecular weight and has 53 amino acid residues and three intramolecular disulfide bonds. For example, EGFs are commercially available (e.g., Corning). For example, EGF is 5-125ng/ml, 10-60ng/ml, 20-30ng/ml, preferably 25ng/ml when included in a culture medium. EGF is occasionally abbreviated herein as "E."

The term "Rho-kinase (ROCK) inhibitor" herein is, for example, R-(+)-trans-4-(1-aminoethyl)-N-(4-pyridyl)cyclohexanecarboxamide dihydrochloride monohydrate (Y-27632, Sigma-Aldrich), 5-(1,4-diazepan-1-ylsulfonyl)isoquinoline (Fasudil or HA1077, Cayman Chemical), or (S)-(+)-2-methyl-1-[(4-methyl-5-isoquinolinyl) sulfonyl]-hexahydro-1H-1,4-diazepine dihydrochloride (H-1152, Tocris Bioscience). For example, ROCK inhibitors are commercially available. In an aspect, the ROCK inhibitor is Y-27632. The ROCK inhibitor is an amount such that it can exert the inhibition effect corresponding to Y-27632 of 2-50 µM, 4-25 µM, or 8-12 µM, preferably 10 µM when included in a culture medium. T-27632 is occasionally abbreviated herein as "Y."

In an embodiment, the addition agents of the disclosure may be a combination of at least one selected from the group consisting of KGF, FGF10, and HGF; BMP: inhibitor; and TGFβ inhibitor. The addition agents may further comprise either or both of the agent capable of activating Wnt signaling and ROCK inhibitor. In an embodiment, the addition agents may be, for example, a combination of KGF, BMP inhibitor, TGFβ inhibitor; a combination of KGF, the agent capable of activating Wnt signaling, BMP inhibitor, TGFβ inhibitor; a combination of FGF10, BMP inhibitor, and TGFβ inhibitor; a combination of FGF10, the agent capable of activating Wnt signaling, BMP inhibitor, and TGFβ inhibitor; a combination of HGF, BMP inhibitor, and TGFβ inhibitor; a combination of HGF, the agent capable of activating Wnt signaling, BMP inhibitor, and TGFβ inhibitor; a combination of KGF, FGF10, HGF, BMP inhibitor, and TGFβ inhibitor; a combination of KGF, FGF10, HGF, the agent capable of activating Wnt signaling, BMP inhibitor, and TGFβ inhibitor; a combination of ROCK inhibitor, KGF, FGF10, HGF, BMP inhibitor, and TGFβ inhibitor; or a combination of ROCK inhibitor, KGF, FGF10, HGF, the agent capable of activating Wnt signaling, BMP inhibitor, and TGFβ inhibitor. In the addition agents, the BMP inhibitor may be Noggin; the TGFβ inhibitor may be SB431542; the agent capable of activating Wnt signaling may be CHIR99021; and/or the ROCK inhibitor may be Y-27632.

In an embodiment, the culture medium contains "extracellular matrix." The extracellular matrix (ECM) includes, but is not limited to, water, polysaccharides, elastin, integrins, and glycoproteins. Glycoproteins include, for example, collagen, entactin (nidogen), fibronectin, and laminin. For example, the ECM may be prepared by culturing ECM-producing cells (e.g., epithelial cells, endothelial cells, parietal-endoderm-like cells, or fibroblasts) *in vitro* and then removing the ECM-producing cells. The ECM-producing cells may be, for example, cartilagenous cells predominantly producing collagen and chondrocytes; fibroblasts predominantly producing collagen type IV, laminin, stromal procollagen, and fibronectin, and stromal fibroblasts predominantly producing collagen (types I, III, and V), chondroitin sulfate proteoglycans, hyaluronic acid, fibronectin, and tenascin-C. The ECM is commercially available. Commercially available extracellular matrix may be, for example, Extracellular Matrix Protein (invitrogen), basement membrane preparations from Engelbreth-Holm-Swarm (EHS) mouse sarcoma cells (e.g., Cultrex^{®} Basement Membrane Extract (Trevigen, Inc.), or Matrigel^{®} (Corning Inc.)). The ECM may be a synthetic extracellular matrix (e.g., ProNectin (Sigma Z378666)). The extracellular matrix may be one kind or a mixture of two or more kinds. In an embodiment, the ECM is Matrigel. In an embodiment, the culture medium is free of extracellular matrix.

The extracellular matrix may exist as a "dispersed component" when included in a culture medium. For example, the extracellular matrix as a dispersed component is in the form that extracellular matrix components are dispersed or dissolved in culture medium. The extracellular matrix as a dispersed component is included, for example, at 10% v/v or less, 7% v/v or less, 5% v/v or less, e.g., 2.5% v/v per volume of culture medium. In an embodiment, the culture medium contains the extracellular matrix of 0-10%vlv, 0-7%v/v, 0-5%v/v, 0.1-10%v/v, 0.1-7%v/v, 0.1-5%v/v, 1-10%v/v, 1-7%v/v, 1-5%v/v, 1-2.5%v/v, 2-10%v/v, 2-7%v/v, 2-5%v/v, or 2-2.5%v/v in amount. Suppose the extracellular matrix contains preparations of biological origin (e.g., basement membrane preparations from mouse sarcoma cells). In that case, the extracellular matrix is preferably at low concentrations in the culture medium in view of the prevention or reduction of contamination of organisms formed.

The extracellular matrix may exist as a "scaffold" when included in a culture medium. For example, the extracellular matrix as a scaffold is the form to provide cells with microenvironments that at least partially resemble the cellular niche in which cells are present in nature. The extracellular matrix as a scaffold may be, for example, in gel form. The extracellular matrix as a scaffold may be, for example, gel formed of more than 10% v/v, not less than 20% v/v, not less than 30% v/v, not less than 50% v/v, or not less than 70% v/v in amount.

The term "feeder cell" herein refers to a cell type used to assist stem cells in proliferating. Feeder cells are mainly used to provide surfaces suitable for assisting cells of interest in proliferating by co-culturing with the cells of interest. Feeder cells are cells themselves isolated from a specific cell type (e.g., primary adult mouse fibroblasts, mouse fibroblast cell line (MLg), and human fetal fibroblast cell line: MRC-5). Feeder cells are prepared by administering antibiotics or irradiating gamma lay so that cells are not lethal in advance. The use of feeder cells is undesirable because it complicates the passaging of cells of interest. In an embodiment, the culture medium is substantially free of feeder cells. The term "substantially free" herein does not exclude " entirely free." In an embodiment, a culture medium substantially free of feeder cells does not entirely include feeder cells. in an embodiment, the culture medium substantially free of feeder cells may include less than 3%, less than 2%, less than 1%, or less than 0.5% of feeder cells relative to the number of cells to be cultured, the cells including lung epithelial cells or lung cancer cells to be cultured. Preferably, the culture medium including lung epithelial cells or lung cancer cells to be cultured does not include feeder cells.

The methods disclosed in Patent Literature2 may be used as a method for "culturing" a sample including lung epithelial cells or lung cancer cells in a culture medium. The culturing method may include, for example, introducing a sample including lung epithelial cells or lung cancer cells into a culture medium in a culture vessel and keeping the culture medium at 37°C under 5% CO₂. The temperature for culturing is not limited to 37°C, but any temperature known in the field of cell culture may be appropriately used. The CO₂ concentration is not limited to 5%, but any CO² concentration known in the field of cell culture may be appropriately used. The culture medium may be exchanged with a new culture medium at any interval, for example, every 1-14 days, every 2-12 days, or every 3-10 days. For example, the culture medium may be exchanged every 3-7 days, every 3-5 days, or every 3 days if the cells to be cultured are lung epithelial cells. For example, the culture medium may be exchanged every 3-14 days, every 5-10 days, or every 10 days if the cells to be cultured are lung cancer epithelial stem cells.

"Organoid derived from lung epithelial cell" or "organoid derived from lung cancer cell" formed by culturing may be isolated from the culture medium. Isolation may include, for example, fractionating cells based on their size and separating organoids from cells that do not form cell aggregate. For example, isolation can be performed with a CELL HANDLER (Yamaha).

In an embodiment, the method according to the present aspect comprises selecting club-I cell, AT2 cell, or club-II cell from a sample that includes lung epithelial cells prepared from lung tissue of a mammalian, based on at least one cell markers corresponding to respective club-I cell, AT2 cell, and club-II cell listed in Table A and homologs thereof, before culturing the sample in a culture medium containing addition agents of the present disclosure.

### [TABLE 1]

**Table A**

| club-I cell | AT2 cell | club-ll cell |
|---|---|---|
| Cbr2 | Lamp3 | Tff2 |
| Hp | Lyz2 | Reg3g |
| Cyp2f2 | Napsa | Bpifb1 |
| Prdx6 | Slc34a2 | Muc5b |
| Scgb1a1 | Lyz1 | Sult1d1 |
| Ldhb | Rnase4 | Fxyd3 |
| Ces1d | Hc | Scgb3a1 |
| Cckar | Cd74 | Lypd2 |
| Selenbp1 | Scd1 | Chad |
| Gstm2 | Sftpc | S100a6 |
| Scnn1b | Lgi3 | Pglyrp1 |
| Scgb1c1 | Cldn18 | Aldh3a1 |
| Plpp3 | Etv5 | Bpifa1 |
| Ephx1 | Cxcl15 | Gsto1 |
| Dcxr | S100g | Lgals3 |
| Alas1 | Elovl1 | Pigr |
| Retnla | Fas | Scgb3a2 |
| Sec14l3 | H2-Aa | Ltf |
| Ptgr1 | Fabp5 | Qsox1 |
| Krtap17-1 | Rn4.5s | Cyp2a5 |
| | | Cpd |
| | | Ly6a |
| | | Perp |
| | | Kcne3 |
| | | Il13ra1 |
| | | Slc15a2 |
| | | Cd14 |

In an embodiment, the method comprises selecting club-I cells from the sample based on at least one of the cell markers for club-I cell listed in Table A and homologs thereof. In an embodiment, the method comprises selecting AT2 cells from the sample based on at least one of the cell markers for AT2 cell listed in Table A and homologs thereof. In an embodiment, the method comprises selecting club-II cells from the sample based on at least one of the cell markers for club-II cell listed in Table A and homologs thereof.

The term "club-I cell" herein refers to a club cell that expresses at least one of the cell markers listed in Table A and homologs thereof. For example, Club-I cells form organoids in a culture medium containing addition agents of the present disclosure. For example, club-I cells express at least one, at least two, at least three, or at least four cell markers selected from the group consisting of Cbr2, Hp, Cyp2f2, Prdx6, Scgb1a1, Ldhb, Ces1d, Cckar, Se!enbp1, Gstm2, Scnn1b, Scgb1c1, Plpp3, Ephx1, Dcxr, Alas1, Retnla, Sec14l3, Ptgr1, and Krtap17-1 and homologs thereof.

The term "club-II cell" herein refers to a club cell that expresses at least one of the cell markers listed in Table A and homologs thereof. For example, club-II cells form organoids in a culture medium containing addition agents of the present disclosure. For example, club-II cells tend to form organoids more than club-I cells. For example, club-II cells express at least one, at least two, at least three, or at least four cell markers selected from the group consisting of Tff2, Reg3g, Bpifb1, Muc5b, Sult1d1, Fxyd3, Scgb3a1, Lypd2, Chad, S100a6, Pglyrp1, Aldh3a1, Bpifa1, Gsto1, Lgals3, Pigr, Scgb3a2, Ltf, Qsox1, Cyp2a5, Cpd, Ly6a, Perp, Kcne3, Il13ra1, Slc15a2, and Cd14 and homologs thereof. For example, club-II cells express at least one, at least two, at least three, or at least four cell markers selected from the group consisting of Fxyd3, Pigr, Cpd, Ly6a, Perp, Kcne3, Il13ra1, Slc15a2, and Cd14 and homologs thereof.

The term "AT2 cell" or "alveolar epithelial type 2 cell," according to embodiments in which predetermined cells are selected based on cell markers, refers to an AT2 cell expressing at least one of the cell markers listed in Table A and homologs thereof. For example, AT2 cells form organoids in a culture medium containing addition agents of the present disclosure. For example, AT2 cells express at least one, at least two, at least three, or at least four cell markers selected from the group consisting of Lamp3, Lyz2, Napsa, Slc34a2, Lyz1, Rnase4, He, Cd74, Scd1, Sftpc, Lgi3, Cldn18, Etv5, Cxcl15, S100g, Elovl1, Fas, H2-Aa, Fabp5, and Rn4.5s and homologs thereof.

To "select" predetermined cells herein means to choose predetermined cells from a sample including various types of cells. For example, selecting predetermined cells can be performed by measuring the expression of predetermined cell markers (e.g., cell markers listed in Table A) from the sample containing various types of cells and fractionating the cells expressing the predetermined cell markers as the predetermined cells based on the measurement results. For example, selecting predetermined cells can be performed by fluorescence-activated cell sorting (FACS). FACS can be performed, for example, with a commercially available FACS device in which a fluorescent probe is used. The fluorescent probe is a probe (e.g., antibody) capable of binding to a given cell marker conjugated with fluorophore. In one example, when the expression level of the predetermined cell markers in subject cells is higher than the expression level of the predetermined cell markers in the negative control, the subject cells can be selected as the predetermined cells. For example, fluorophor may be prepared according to known methods or commercially available. For example, probes, e.g., antibodies binding to a predetermined cell marker, can be prepared according to known methods or commercially available. For example, the conjugation of fluorophor to probes can be performed according to known methods. In an embodiment, selecting predetermined lung epithelial cells can be performed based on the expression of at least one of cell markers listed in Table A and homologs thereof.

The term "homolog" herein refers to a gene or protein whose sequence and function are similar to those between the same and different species. Homologs herein include orthologs, paralogs, and xenologs. For example, the "homolog" of a particular gene or protein has at least 75%, 80%, 85%, or 90% homology to the particular gene or protein, preferably at least 95%, 96%, 97%, 98%, or 99% homology thereto. Homology refers to a percentage of identical nucleotide or amino acid residues between two gene or protein sequences after being aligned by an alignment algorithm known to those skilled in the art. For example, homology can be performed by known methods or publicly available computer programs (e.g., BLAST or FASTA).

The term "Cbr2" herein refers to carbonyl reductase 2, which is involved in the synthesis of NADPH.

The term "Hp" herein refers to haptoglobin, which is a hemoglobin-binding protein.

The term "Cyp2f2" herein refers to cytochrome P450(CYP)2f2, which is a mouse-specific membrane-bound protein that localizes on the endoplasmic reticulum membrane. Homologs of Cyp2f2 in mammals other than mouse are known. For example, CYO2F1 is the homolog in humans.

The term "Prdx6" herein refers to peroxiredoxin -6, which is a protein belonging to the peroxiredoxin family of antioxidant enzymes. The protein encoded by PDRX6 gene is the homolog in humans.

The term "Scgb1a1" herein refers to a member of secretoglobulin family 1A, which possesses an anti-inflammatory effect.

The term "Ldhb" herein refers to lactate dehydrogenase B, which is a monomer of the LDH enzyme encoded by LDHB gene.

The term "Ces1d" herein refers to a carboxylesterase 1d.

The term "Cckar" herein refers to a cholecystokinin A receptor, which is the subtype A of two distinct subtypes of G protein-coupled receptors bound by peptide hormones.

The term "Selenbp1" herein refers to selenium-binding protein 1, which is a protein belonging to the selenium-binding protein family. Selenbp1 is encoded by SELENBP1 gene in humans.

The term "Gstm2" herein refers to glutathione S-transferase Mu2, which is the enzyme encoded by GSTM2 gene in humans.

The term "Senn1b" herein refers to sodium channel subunit beta 1, which is the protein encoded by SCN1B gene in humans.

The term "Scgb1c1" herein refers to secretoglobin 1C1, also known as ligand-binding protein RYD5 Scgb1c1. Scgb1c1 is encoded by secretoglobin 1C 1 gene.

The term "Plpp3" herein refers to phospholipid phosphatase 3, also known as phosphatidic acid phosphatase type 2B. Plpp3 is the enzyme encoded by PPAP2B gene on chromosome 1 in humans.

The term "Ephx1" herein refers to epoxide hydrolyzing enzyme 1, which is the enzyme encoded by EPHX1 gene in humans.

The term "Dcxr" herein refers to dicarbonyl L dicarbonyl, which is a multifunctional protein involved in various protein-protein interaction processes in various physiological systems. The term "Alas1" herein refers to a protein known as delta-aminolevulinic acid synthase 1, which is encoded by ALAS1 gene in humans.

The term "Retnla" herein refers to a resistin-like molecule alpha, which is a protein belonging to a family of small cysteine-rich secreted proteins.

The term "Sec14l3" herein refers to SEC14-like 3, which is also known as tocopherol-associated protein 2 and is a phosphatidylinositol transfer protein that plays an important role in the biogenesis of Golgi-derived transport vesicles.

The term "Ptgr1" herein refers to prostaglandin reductase 1, which is a protein involved in the catabolism of eicosanoids and the lipid peroxidation of 4-hydroxynonenal and so on. The term "Krtap17-1" herein refers to keratin-associated protein 17-1, which is a protein belonging to the keratin-associated protein (KAP) family.

The term "Tff2" herein refers to Trefoil Factor 2, which is the protein encoded by TFF2 gene in humans.

The term "Reg3g" herein refers to Regenerating islet-derived protein 3 gamma, which is a protein encoded by REG3G gene in humans.

The term "Bpifb1" herein refers to BPI Fold Containing family B member 1, which is the protein encoded by BPIFB1 gene in humans.

The term "Muc5b" herein refers to mucin 5 subtype B, which is the protein encoded by MUC5B gene in humans.

The term "Sult1d1" herein refers to sulfotransferase 1D member 1, which transfers sulfate groups to hormones and neurotransmitters. It is known to be pseudogenic by a mutation in humans.

The term "Fxyd3" herein refers to FXYD domain-containing ion transport regulator 3, which is the protein encoded by FXYD3 gene in humans.

The term "Scgb3a1" herein refers to secretoglobin family 3A member 1, which is the protein encoded by SCGB3A1 gene in humans.

The term "Lypd2" herein refers to Ly6/PLAUR domain-containing protein 2, which is the protein encoded by LYPD1 gene in humans.

The term "Chad" herein refers to cadherin, which is a transmembrane glycoprotein involved in adhesion between animal cells.

The term "S100a6" herein refers to S100 calcium-binding protein A6, which is the protein encoded by S100A6 gene in humans.

The term "Pgiyrp1" herein refers to peptidoglycan recognition peptidoglycan protein 1, which is the protein encoded by PGLYRP1 gene in humans.

The term "Aldh3a1" herein refers to aldehyde dehydrogenase 3 family member A1, which is the protein encoded by ALDH3A1 gene in humans.

The term "Bpifa1" herein refers to palate, lung, and nasal epithelium clone protein (PLUNC), which is the protein encoded by Bactericidal/permeability-increasing fold-containing family A1 (BPIFA1) gene.

The term "Gsto1" herein refers to glutathione S transferase omega-1, which is the protein encoded by GSTO1 gene in humans.

The term "Lgals3" herein refers to galectin-3, which is the protein encoded by the LGALS3 gene in humans and belongs to a lectin family.

The term "Pigr" herein refers to macromolecular immunoglobulin receptor, which is the transmembrane protein encoded by PIGR gene in humans.

The term "Scgb3a2" herein refers to secretoglobin family 3A member 2, which is the protein encoded by SCGB3A2 gene in humans

The term "Ltf" herein refers to lactoferrin, which is an iron-binding glycoprotein mainly found in mammal milk.

The term "Qsox1" herein refers to sulfhydryl oxidase 1, which is the protein encoded by QSOX1 gene in humans.

The term "Cyp2a5" herein refers to cytochrome P450(CYP)2A5, which is a protein expressed in the olfactory epithelium of the liver and nasal cavity in mice. Homologs or orthologs of Cyp2a5 in mammals other than mouse are known. For example, the orthologue is CYP2A6/13 in humans and CYP2A3 in rats.

The term "Cpd" herein refers to carboxypeptidase D, which is the protein encoded by CPD gene in humans.

The term "Ly6a" herein refers to lymphocyte antigen 6 complex, locus A, which is also referred to as Sca-1 and is a glycosylphosphatidylinositol (GPI)-anchored protein.

The term "Perp" herein refers to p53 apoptosis effector related to PMP-22, which is the membrane protein encoded by PERP gene in humans.

The term "Kcne3" herein refers to potassium voltage-gated channel, Isk-related family, member 3, which is also referred to as MinK-related peptide 2 (MiRP2). Kcne3 is the protein encoded by KCNE3 gene in humans.

The term "Il13ra1" herein refers to interleukin-13 receptor subunit alpha 1, which forms a heterodimer with interleukin-4 receptor alpha and functions as a receptor for interleukin-13.

The term "Slc15a2" herein refers to solute carrier family 15, member 2, which is referred to as a proton-binding peptide transporter.

The term "Cd14" herein refers to CD14 protein, which functions as a component used in the innate immune system. CD14 can be, for example, a membrane-bound protein, mCD14, or a soluble protein, sCD14. For example, CD14 is mCD14 in the context of selecting club-II cells.

The term "Lamp3" herein refers to lysosome-associated membrane glycoprotein 3, which is the protein encoded by LAMP3 gene in humans.

The term "Lyz2" herein refers to lysozyme C-2.

The term "Napsa" herein refers to napsin A, which is the aspartate proteinase encoded by NAPSA gene in humans.

The term "Slc34a2" herein refers to sodium-dependent phosphate transport protein 2B (NaPi2b), which is the protein encoded by SLC34 A2 gene in humans.

The term "Lyz1" herein refers to lysozyme C-1.

The term "Rnase 4" herein refers to ribonuclease 4, which is the protein encoded by RNASE4 gene in humans.

The term "Hc" herein refers to hemolytic complement, which is a component of the complement system in innate immunity.

The term "Cd74" herein refers to HLA class II histocompatibility antigen gamma chain, which is the protein encoded by CD74 gene in humans.

The term "Scd1" herein refers to stearoyl-CoA desaturase-1, which is an enzyme important for fatty acid metabolism.

The term "Sftpc" herein refers to surfactant protein C (SP-C), one of the lung's surfactant proteins. Sftpc is encoded by SFTPC gene in humans.

The term "Lgi3" herein refers to Leucine-rich glioma inactivated 3, which is a secreted protein belonging to the LGI family in vertebrates.

The term "Cldn18" herein refers to claudin 18, which is the protein encoded by CLDN18 gene in humans.

The term "Etv5" herein refers to Ets variant 5, which is also referred to as ERM transcription factor. Etv5 is the transcription factor encoded by ETV5 gene in humans.

The term "Cxcl15" herein refers to chemokine (C-X-C motif) ligand 15, which is a cytokine belonging to the CXC chemokine family.

The term "S100g" herein refers to S100 calcium-binding protein G, which is the protein encoded by S100G gene in humans.

The term "Elovl1" herein refers to fatty acid elongase 1, which is an enzyme that plays an important role in the production of saturated and monounsaturated long-chain fatty acids. The term "Fas" herein refers to fatty acid synthase, which is the enzyme encoded by FASN gene in humans.

The term "H2-Aa" herein refers to histone H2A type 1-A, which is the protein encoded by HIST1H2AA gene in humans.

The term "Fabp5" herein refers to epidermal-type fatty acid binding protein, which is the protein encoded by the FABP5 gene in humans.

The term "Rn4.5s" herein refers to 4.5S RNA.

In an embodiment, the method of the aspect comprises further culturing, in a culture medium not containing addition agents of the present disclosure, lung epithelial cells cultured in a culture medium containing the addition agents. In the embodiment, the lung epithelial cells cultured in the culture medium may form cell aggregates smaller than 50 µm in diameter or form organoids. The base lung epithelial cells cultured in the culture medium preferably form organoids. Further culturing, in a culture medium not containing the addition agents, the lung epithelial cells cultured in the culture medium containing the addition agents of the present disclosure may lead to differentiation of the cultured lung epithelial cells, e.g., from club cells to alveolar epithelial cells (e.g., AT2 cells, AT1 cells).

The culture medium not containing addition agents of the present disclosure may be, for example, a basal medium. Culturing the cells in the culture medium containing the addition agents may comprise, for example, passaging the cultured cells. Culturing the cells in the culture medium not containing the addition agents may comprise, for example, passaging the cultured cells. The days of culturing the cells in the culture medium may be, for example, 1-30 days, 1-25 days, or 1-20 days.

For example, organoids derived from lung epithelial cells may imitate a general physiological function of the lung epithelial tissue. Accordingly, the organoids derived from lung epithelial cells are useful in regenerative medicine. In addition, organoids derived from lung cancer cells are useful in screening for substances capable of treating lung cancer (NATURE COMMUNICATIONS 2019 103991).

### [Organoid derived from lung epithelial cell or lung cancer cell)

An aspect of the present disclosure is to provide an organoid derived from a lung epithelial cell or lung cancer cell. The organoid may be produced from a sample including the lung epithelial cell or lung cancer cell by the method of the present disclosure. The produced organoid derived from the lung epithelial cell or lung cancer cell comprises, for example, an intracavity and a cell layer facing the intracavity.

In an embodiment, an organoid derived from a lung epithelial cell comprises an intracavity and a cell layer facing the intracavity. In one example, the organoid derived from the lung epithelial cell may be organoids derived from lung epithelial cells or organoids derived from airway cells. In one example, the organoid derived from the lung epithelial cell comprises an intracavity and a cell layer facing the intracavity, wherein the cell layer consists essentially of AT2 cells. In the example, the AT2 cells express, for example, either or both of HT2-280 and SFTPC, and the HT2-280 locally localizes in or near cell membranes of the AT2 cells facing the intracavity

In other examples, an organoid derived from a lung epithelial cell comprises an intracavity and a cell layer facing the intracavity, wherein the cell layer consists essentially of a combination of AT2 cells and the cells expressing markers for AT2 cell and bronchial epithelium. In another example, the organoid derived from the lung epithelial cell comprises an intracavity and a cell layer facing the intracavity, wherein the cell layer consists essentially of cells expressing a marker for bronchial epithelium. In one example, the organoid derived from an airway cell comprises an intracavity and a cell layer facing the intracavity, wherein the cell layer consists essentially of basal cells and cells expressing markers for basal cell and bronchial epithelium.

The marker for AT2 cell may use, for example, any known marker for AT2 cell. The marker for AT2 cell may be, for example, either or both of HT2-280 and SFTPC. The marker for bronchial epithelium may use, for example, any known marker for bronchial epithelium. The marker for bronchial epithelium may be, for example, SOX2. The marker for basal cell may use, for example, any known marker for basal cell. The marker for basal cell may be, for example, KRT5.

The term "HT2-280" herein refers to a protein of 280-300 kDa, expressed in alveolar epithelial type 2 cell. The term "SFTPC" herein refers to lung surfactant protein C. SFTPC has a hydrophobic α-helix structure connected to 9 hydrophilic amino acids, the α-helix structure consisting of 26 amino acids. The term "SOX2" herein refers to SRY-box containing gene 2. SOX2 consists of a DNA binding site, the HMG (high mobility group) domain, and its C-terminal transcriptional activation domain. The HMG domain contains two nuclear localization signal sequences, and SOX2 localizes in nuclear. The term "KRT5" herein refers to the intermediate filament protein encoded by KRT5 gene in humans.

### [Regenerative medical composition]

An aspect of the present disclosure is to provide a regenerative medical composition. In an embodiment, the regenerative medical composition comprises an organoid derived from a lung epithelial cell according to the present disclosure and/or any organoid described above. In an embodiment, the regenerative medical composition comprises lung epithelial cells consisting essentially of club-I cells, AT2 cells, or club-II cells expressing at least one of the cell markers listed in Table A and homologs thereof.

### [TABLE 2]

**Table A**

| club-I cell | AT2 cell | club-II cell |
|---|---|---|
| Cbr2 | Lamp3 | Tff2 |
| Hp | Lyz2 | Reg3g |
| Cyp2f2 | Napsa | Bpifb1 |
| Prdx6 | Slc34a2 | Muc5b |
| Scgb1a1 | Lyz1 | Sult1d1 |
| Ldhb | Rnase4 | Fxyd3 |
| Ces1d | He | Scgb3a1 |
| Cckar | Cd74 | Lypd2 |
| Selenbp1 | Scd1 | Chad |
| Gstm2 | Sftpc | S100a6 |
| Scnn1b | Lgi3 | Pglyrp1 |
| Scgb1c1 | Cldn18 | Aldh3a1 |
| Plpp3 | Etv5 | Bpifa1 |
| Ephx1 | Cxcl15 | Gsto1 |
| Dcxr | S100g | Lgals3 |
| Alas1 | Elovl1 | Piqr |
| Retnla | Fas | Scgb3a2 |
| Sec1413 | H2-Aa | Ltf |
| Ptqr1 | Fabp5 | Qsox1 |
| Krtap17-1 | Rn4.5s | Cyp2a5 |
| | | Cpd |
| | | Ly6a |
| | | Perp |
| | | Kcne3 |
| | | Il13ra1 |
| | | Slc15a2 |
| | | Cd14 |

The regenerative medical composition may be, for example, a pharmaceutical composition for repairing lung injury or lung disease. In an embodiment, the regenerative medical composition comprising lung epithelial cells consisting essentially of club-I cells, AT2 cells, or club-II cells expressing at least one of the cell markers described above may be a pharmaceutical composition containing the addition agents of the present disclosure. In one example, the regenerative medical composition comprising lung epithelial cells comprising or consisting essentially of club-I cells is useful as a regenerative medical composition for repairing lung injury or lung disease, because the club-I cells possess the capability to form organoids and to differentiate into cells of the pulmonary alveoli and bronchus lineage without causing inflammation or other adverse effects in the lung to which they are delivered.

In one example, the regenerative medical composition comprising lung epithelial cells comprising or consisting essentially of club-II cells may be useful as a pharmaceutical composition for treating lung injury or lung disease by using the club-II cells as carriers for a drug (e.g., organic compound, gene, or protein) for treating damaged or diseased lung. Accordingly, an aspect of the present invention is to provide a pharmaceutical composition comprising a drug for lung injury or lung disease and lung epithelial cells consisting essentially of club-II cells, the club-II cells being selected from a sample including lung epithelial cells based on the cell markers for club-II cell listed in Table A and homologs thereof, the sample being prepared from mammalian lung tissue.

The regenerative medical composition may comprise, for example, a pharmaceutically acceptable carrier. The regenerative medical composition may be produced according to known methods. The regenerative medical composition may be produced, for example, by mixing organoids derived from lung epithelial cells and/or cells of the organoids with a pharmaceutically acceptable carrier. The term "pharmaceutically acceptable carrier" refers to any component other than organoids derived from lung epithelial cells of the present disclosure, the component being safe in mammals and having low allergic reactivity. The pharmaceutically acceptable carrier includes, for example, an aqueous or non-aqueous solvent (e.g., saline, basal medium, or cell suspension preservative solution) suitable for administering medicament, a cryoprotectant (e.g., glycerol), a water-soluble polymer (e.g., dextran), or buffer (e.g., phosphate buffer).

The organoid derived from lung epithelial cells can be prepared according to the production method in the present disclosure. The organoid derived from the lung epithelial cells comprises an intracavity and a cell layer facing the intracavity. The "cells of organoids derived from lung epithelial cells" may be cells in the form of aggregate constituting the organoid derived from the lung epithelial cells and/or cells in the form of individually dispersed from the organoid derived from the lung epithelial cells. The cells in the form of individually dispersed from the organoid derived from the lung epithelial cells may be prepared, for example, by treating the organoid with protease (e.g., collagenase, dispase, elastase or trypsin) and then suspending it in a predetermined solution (e.g., basal medium). When the regenerative medical composition comprises cells in the form of aggregate constituting the organoid derived from lung epithelial cells, the cell aggregates in the regenerative medical composition may be treated, for example, to make it individual cells before administration to a mammal in need thereof.

For example, the regenerative medical composition is administered to a mammal in need thereof by surgical transplantation into a predetermined site or injection into a predetermined site. In view of reduced graft rejection, the mammal to which the regenerative medical composition is administered is preferably the same species and is more preferably the same individual as the mammal that provided lung epithelial cells to form the organoids. In the context of regenerative medical composition, a mammal is, for example, a human.

The term "lung injury" or "lung disease" herein refers to, for example, pathological disease, lung cancer (e.g., small cell or non-small cell lung cancer (e.g., adenocarcinoma, squamous cell carcinoma, or large cell carcinoma)), interstitial lung disease, pneumonia (e.g., organizing pneumonia), tuberculosis, cystic fibrosis, bronchitis, pulmonary fibrosis, sarcoidosis, hyperplasia of type II, chronic obstructive lung disease, emphysema, asthma, pulmonary edema, acute respiratory distress syndrome, stridor, bronchiectasis, Hantavirus pulmonary syndrome, Middle East Respiratory Syndrome (MERS), Severe Acute Respiratory Syndrome (SARS), and pneumoconiosis. Pathological diseases may be, for example, diseases caused by adenovirus, coronavirus (e.g., COVID-19, SARS-CoV, SARS-CoV-2, or MERS-CoV, or mutant strains thereof), Human metapneumovirus, Influenza virus, Parainfluenza virus, Respiratory syncytial virus, Rhinovirus, Hantaviruses, Enteroviruses (e.g., enterovirus D68:EV-D68), Bordetella pertussis, Chlamydophila pneumonia, Diphtheria, Coxiella burnetii, Haemophilus influenzae, Legionella pneumophila, Moraxella catarrhalis, Bacillus tuberculosis, Mycoplasma pneumoniae, Staphylococcus aureus, Streptococcus pneumoniae, or Streptococcus pyogenes.

### [Method of screening a substance capable of treating lung cancer]

An aspect of the present disclosure is to provide a method of screening a substance capable of treating lung cancer. The screening method comprises contacting a subject substance with an organoid derived from a lung cancer cell, produced by a method of the present disclosure, measuring the organoid after contact with the subject substance, and comparing the measured value from the organoid after contact with the subject substance to a control value. In an embodiment, the method comprises determining, based on the compared result, whether the subject substance is a substance capable of treating lung cancer.

The term "lung cancer" herein may be, for example, small cell lung cancer or non-small cell lung cancer (e.g., adenocarcinoma, squamous cell carcinoma, or large cell carcinoma).

The term "subject substance" herein refers to, for example, small molecule compound, protein (e.g., antibody), DNA, RNA, small interfering RNA, or antisense oligonucleotide. For example, the subject substance may be an agent for treating a disease or cancer other than lung cancer. For example, the subject substance may be one or a mixture of two or more kinds. The subject substance is preferably one kind of substance.

To "contact" an organoid derived from lung cancer cells with a subject substance according to the present disclosure means to place the organoid and subject substance in a situation in which they can be contacted. For example, the contact between the organoid and the subject substance may be performed by adding the subject substance to a solution including the organoid.

The term "measuring an organoid" herein may include, for example, measuring the size of the organoid or measuring the expression of a marker protein in cells constituting the organoid. For example, the size of the organoid can be measured with a known device (e.g., microscope or FACS). The expression of a marker protein in cells constituting the organoid can be measured with a known device (e.g., microscope or FACS) and a known reagent (e.g., fluorophor-labeled antibody). For example, the size of the organoid can be the perimeter, diameter (e.g., major axis and minor axis), or area. ), or area of the organoid. For example, the expression of a marker protein in cells constituting the organoid may be the fluorescence intensity corresponding to the amount of marker protein per area of the organoid. The marker protein to be measured may be one or a combination of two or more marker proteins. The marker protein to be measured is preferably a combination of two or more marker proteins.

The term "measured value of an organoid" herein may be, for example, one measured value by one measurement, an average value by multiple measurements, or an average value of measured values from the plural of organoids. The measured value of the organoid may be, for example, the changed value (e.g., difference or multiple) before and after contacting the subject substance.

The term "control value" herein may be, for example, the measured value when an agent for treating lung cancer cells is used as a control substance. For example, when the measured value from an organoid is the measured value from the organoid after contacting a subject substance, a control value may be a measured value from a control organoid, which is the organoid before contacting or not contacting the subject substance.

In the case where the measured value is the size of the organoid, "determining" whether a subject substance is a substance capable of treating lung cancer may comprise, for example, determining that the subject substance is a substance capable of treating lung cancer if the size of the organoid after contacting the subject substance is smaller than the control value.

### (Culture medium]

An aspect of the present disclosure is to provide a culture medium for producing an organoid from lung epithelial cells or lung cancer cells. The culture medium contains 0-10%v/v of extracellular matrix and contains a combination of at least one selected from the group consisting of keratinocyte growth factor (KGF), fibroblast growth factor (FGF) 10, and hepatocyte growth factor (HGF); bone morphogenetic protein (BMP) inhibitor; and TGFβ inhibitor.

For example, the culture medium may be liquid in form, in which each component is blended to the desired concentration. For example, the culture medium may be solid in form, in which each component is blended so as to be the desired concentration by mixing with a predetermined amount of pure water.

Unless otherwise described, explanations of the terms and embodiments herein appropriately apply to the other aspects and embodiments provided by the present disclosure.

The particular examples are described below. However, those examples are merely given to indicate preferred embodiments of the present invention and do not limit the scope of the invention recited in the accompanying claims in a manner.

### EXAMPLES

### [Preparation Example 1]

### (Preparing suspensions including lung epithelial stem cells)

SFTPC-GFP mice were prepared. The mouse includes a gene encoding a green fluorescent protein (GFP) under the control of a human lung surfactant protein C (SFTPC) promoter and expresses GFP along with Sftpc expression (J Immunol 2008;180: 881-888 and Rapid Communications: L349-L356). Tissue sections of peripheral lung regions of the SFTPC-GFP mice were immunostained for Sftpc and Scgb1a1 (also known as CC10 or CCSP).

Scgb1a1 is known as a cell marker of club cells. The above Immunostaining showed that AT2 cells expressing Sftpc and GFP existed in the pulmonary alveolar region and that bronchioalveolar stem cells (BASCs) expressing Sftpc, GFP, and Scgb1a1, and club cells (also known as variant club cells) weakly expressing Sftpc and GFP and expressing Scgb1a1 existed in the peripheral of bronchi. The expression pattern of GFP in the peripheral lung region was similar to that reported in STEM CELLS 2012; 30: 1948-1960. The Immunostaining results indicate that GFP, whose expression is regulated under the SFTPC promoter, may be used as an indicator to identify AT2 cells, BASCs, and club cells, which are lung epithelial stem cells, among various cells in the lung tissues of SFTPC-GFP mice.

An SFTPC-GFP mouse was euthanized by carbon dioxide, and the blood was removed. The sternum was removed to expose the lungs and trachea. Applied via the trachea was SURFLO^{®}, through which a protease solution of 1.5 mL was injected into the lungs and trachea. The lungs were removed from the SFTPC-GFP mouse and placed on a Schale into which a protease solution of 2.5 mL was poured. The lungs were cut into small pieces on the Schale and were pipetted to obtain a lung tissue suspension. The lung tissue suspension was centrifuged (400g × 5 min) at 4°C and separated into precipitate and supernatant, and the supernatant was discarded. The precipitate was mixed with a PBS containing 3% FBS to obtain a suspension including lung epithelial stem cells.

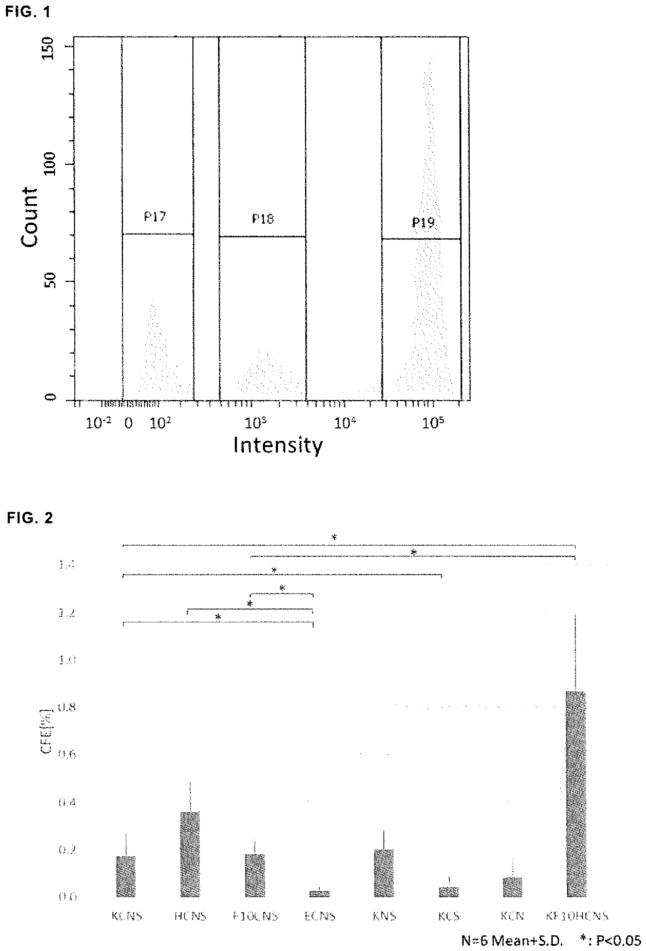

The suspension including lung epithelial stem cells was mixed with anti-EpCAM antibody labeled with fluorophors. Epithelial Cell Adhesion Molecule (EpCAM) is a type I transmembrane glycoprotein known as an intercellular cell adhesion molecule specific to epithelial cells. FACS Aria II (BD) was used to identify lung epithelial stem cells based on the fluorescence intensities derived from anti-EpCAM antibodies. The identified lung epithelial stem cells were further separated into three fractions, P17, P18, and P19, based on their GFP-derived fluorescence intensities (Fig. 1).

### [Test Example 1]

Expression patterns of marker genes were examined to identify cell types in each fraction. The expression patterns of marker genes were examined by amplifying each marker gene by qPCR. The result showed that the P17 fraction predominantly included club cells, Ciliated cells, Type I alveolar epithelial cells (AT1 cells), and basal cells. It also showed that the P18 fraction included variant club (distal club) cells, and the P19 fraction included AT2 cells and BASCs.

### (Basal Medium)

The P19 fraction suspension including lung epithelial stem cells, the P18 fraction suspension including lung epithelial stem cells, and the P17 fraction suspension including lung epithelial stem cells, shown in Figure 1, were cultured without feeder cells to form organoids. MTECIB27 was used as a basal medium for the culturing. The components of MTEC/B27 are described below.

**[TABLE 3]**

| Components | Distributor | Volume (mL) |
|---|---|---|
| DMEM/F12 NO PHENOL RED | Life technologies | 500 |
| 1M HEPES | Nacalai tesque | 7.5 |
| 7.5% NaHCO₃ | Nacalai tesque | 2 |
| B27^{®} Supplement (×50) _{serum-free} | GIBCO | 10 |
| FBS (final concentration 5%) | SIGMA | 25 |
| fungizone (final concentration 250ng/mL) | ThermoFisher | 500 [µL] |
| Penicillin/streptomycin (final concentration 100U/mL, 100µg/mL) | Nacalai | 5 |

### (Addition Agent)

The basal medium was appropriately supplemented with addition agents described below to make a culture medium.

**[TABLE 4]**

| Abbreviatio n | Name | Distributor | Final concentration | Remarks column |
|---|---|---|---|---|
| K | KGF/Fgf7 mouse recombinant | R&D | 50 ng/mL | Fgf7, Fgf ligand |
| C | CHIR99021 | SIGMA | 3 µM | GSK-3 inhibitor (Wnt activator) |
| N | Recombinant murine Noggin | PeproTech | 100 ng/mL | Bmp inhibitor |
| S | TGF-BETA RI KINASE INHIBITOR VI SB431542 | Calbiochem | 10 µM | ALK4.5 inhibitor (Tgfβ signal inhibitor) |
| H | HGF mouse recombinant | R&D | 30 ng/mL | Met ligand |
| F10 | Recombinant Human FGF-10 | PEPROTEC H | 50 ng/mL | Fgf ligand |
| E | EGF | Corning | 25 ng/mL | Egf ligand |
| X | XAV939 | SIGMA | 10 µM | Tankyrase inhibitor(Wnt/βcaten in inhibitor) |
| Y | Y-27632, Dihydrochloride Salt | LC Laboratories | 10 µM | Rock inhibitor |

### (Formation of Organoids from lung epithelial stem cells)

The suspension including lung epithelial stem cells was subjected to centrifugation (400g, 10 min, 4°C), and the precipitate was suspended in MTEC/B27. The lung epithelial stem cells at a predetermined number were mixed with 50% or 75% Matrigel^{®} (Matrigel Matrix basement membrane growth factor reduced (Corning Corp.)). The obtained mixture of 20 µL was dropped onto each well of a 48-well plate heated to 37°C. The plate was kept at 37°C for 20 minutes to form a gel in each well. A culture medium of 250 µL, the basal medium supplemented with a combination of Hgf, Fgf10, KGF, Noggin, and SB431542, was applied to the formed gel. The plate was incubated at 37°C for 6 days under 5% CO₂. The culture medium was exchanged once every 3 days.

Each of the P17 to P19 fractions' suspensions including the lung epithelial stem cells was cultured as described above. As a result, cell aggregates in diameter of not less than 50 µm were observed with a microscope. The cultured products were immunohistochemically stained. The immunohistochemical staining indicated that the observed cell aggregates from the P19 fraction were organoids expressing Sftpc, consisting predominantly of AT2 cells. It indicated that the observed cell aggregates from the P18 fraction were organoids consisting predominantly of cells expressing Sftpc and Scgb1a1, like variant club cells. It indicated that the observed cell aggregates from the P17 fraction were organoids highly expressing Scgb1a1, which were thought to be derived from club cells, and organoids consisting predominantly of cells expressing Krt5, and they were formed, respectively.

### [Test Example 2]

Suspensions including lung epithelial stem cells were prepared from each of six SFTPC-GFP mice (n=6) as described in Preparation Example 1. The fraction corresponding to the P19 fraction, shown in the scatter plot of lung epithelial stem cells plotted according to their GFP fluorescence intensities in Figure 1, is hereinafter referred to as GFP^{hi} fraction. Like this, the fraction corresponding to the P18 fraction, whose GFP fluorescence intensities are lower than those of the P19 fraction, is referred to as GFP^{lo} fraction. The fraction corresponding to the P17 fraction, whose GFP fluorescence intensities are lower than those of the P18 fraction, is referred to as GFP^{neg} fraction.

### (GFP^{hi} Fraction)

GFP^{hi} fraction suspension including lung epithelial stem cells was cultured as in Test Example 1. In Test Example 2, the following combination of addition agents was added to the culture medium, and the suspension including one type of lung epithelial stem cell was cultured in 4 wells, which included 5,000 cells per well, unlike Test Example 1.

**TABLE 5]**

| | Name | **KCNS** | **HCNS** | **F10CNS** | **KNS** | **KCS** | **KCN** | **KF10HCNS** | **ECNS** |
|---|---|---|---|---|---|---|---|---|---|
| **K** | KGF | + | - | - | + | + | + | + | - |
| **C** | CHIR99021 | + | + | + | - | + | + | + | + |
| **N** | Noggin | + | + | + | + | - | + | + | + |
| **S** | SB431542 | + | + | + | + | + | - | + | + |
| **H** | HGF | - | + | - | - | - | - | + | - |
| **F10** | FGF10 | - | - | + | - | - | - | + | - |
| **E** | EGF | - | - | - | - | - | - | - | + |

| | | | | | | | | | |
|---|---|---|---|---|---|---|---|---|---|
| +; Added as an addition agent -: Not added as an addition agent | | | | | | | | | |

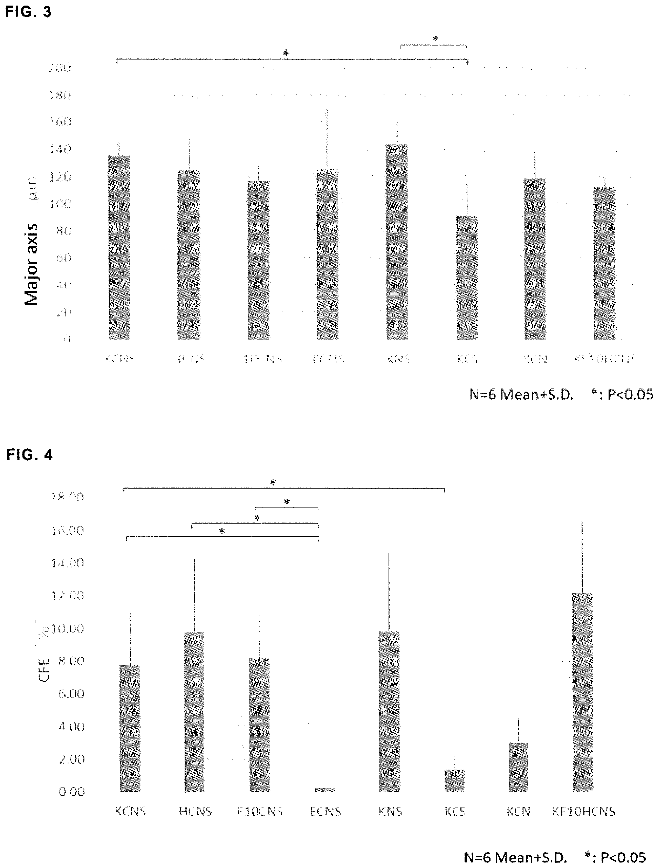

After culturing for 6 days, the cultured products were imaged with a microscope, and the cell aggregates in diameter of not less than 50 µm were counted using a software program. The efficiency of forming organoids was measured by CFE [%], a percentage obtained by dividing the number of cell aggregates in each well by the number of added cells. The average of CFEs (n=6) and its standard deviation were calculated from the CFEs obtained with 6 mice (FIG. 2). The average of the major axis [µm] (n=6) and its standard deviation were calculated from the diameters of organoids obtained with the 6 mice (FIG. 3).

### - Combinations of Four Addition Agents

Figures 2 and 3 show that combining four addition agents, "KCNS," resulted in a CFE of about 0.18% and a major axis of about 136 µm, respectively. The four addition agents' combination "HCNS," in which the addition agent H replaced K in KCNS, indicated the CFE and major axis, respectively, comparable to the "KCNS." The result suggests that the addition agent "K" in the four addition agents' combination KCNS is substitutable for "H" regarding the efficient organoid formation.

The addition agents' combination "F10CNS," in which the addition agent F10 replaced K in "KCNS," indicated the CFE and major axis, respectively, comparable to the "KCNS." The result suggests that the addition agent "K" in the addition agents' combination "KCNS" is substitutable for "F10" regarding the efficient organoid formation. The addition agents' combination "ECNS," in which the addition agent "E" replaced "K" in "KCNS," was significantly lower in CFE than the "KCNS" (Tukey test, *: P<0.05).

### - Combinations of Three Addition Agents

It is examined whether the three addition agents other than addition agent "K," which is substitutable for addition agent "H" or "F10" in the four addition agents' combination "KCNS," play an important role in efficient formation (high CFE) and major axis of organoids. The addition agents' combination "KCS," which removed the addition agent "N" from the "KCNS," was significantly lower in CFE and major axis than the "KCNS," respectively. (Tukey test, *: P<0.05) The addition agents' combination "KCN," which removed the addition agent "S" from "KCNS," was not significantly different in CFE from the "KCNS" but tended to be lower. The results indicate that the addition agents "N" and "S in the four addition agents' combination "KCNS" are important for efficient organoid formation.

The addition agents' combination "KNS," which removed the addition agent "C" from the "KCNS," was not significantly different in CFE or major axis from the "KCNS." The result suggests that the addition agent "C" in the four addition agents' combination "KCNS" is not essential for efficient organoid formation. On the other hand, the addition agent "C" is indicated to induce the differentiation of lung epithelial stem cells, which constitute cell aggregates, as demonstrated in Test Example 3.

### - Combination of Six Addition Agents

Figures 2 and 3 show that combining six addition agents, "KF10HCNS," resulted in a CFE of about 0.87% and a major axis of about 113 µm, respectively. The six addition agents' combination "KF10HCNS" was significantly larger in CFE than the four addition agents' combinations "KCNS" and "F10CNS" (Tukey test, *: P<0.05). The "KF10HCNS" showed a major axis comparable to "KCNS," which showed the largest major axis among the four addition agents' combinations. The result suggests that the six addition agents' combination "KF10HCNS" is preferable for efficient organoid formation.

### - Combination of Five Addition Agents

It is suggested, as described above, that the addition agent "C" in the six addition agents' combination "KF10HCNS" was not essential for efficient organoid formation. Accordingly, the five addition agents' combination "HF10KNS," which removed the addition agent "C" from "KF10HCNS," is thought preferable for efficient organoid formation.

### (GFP^{lo} Fraction)

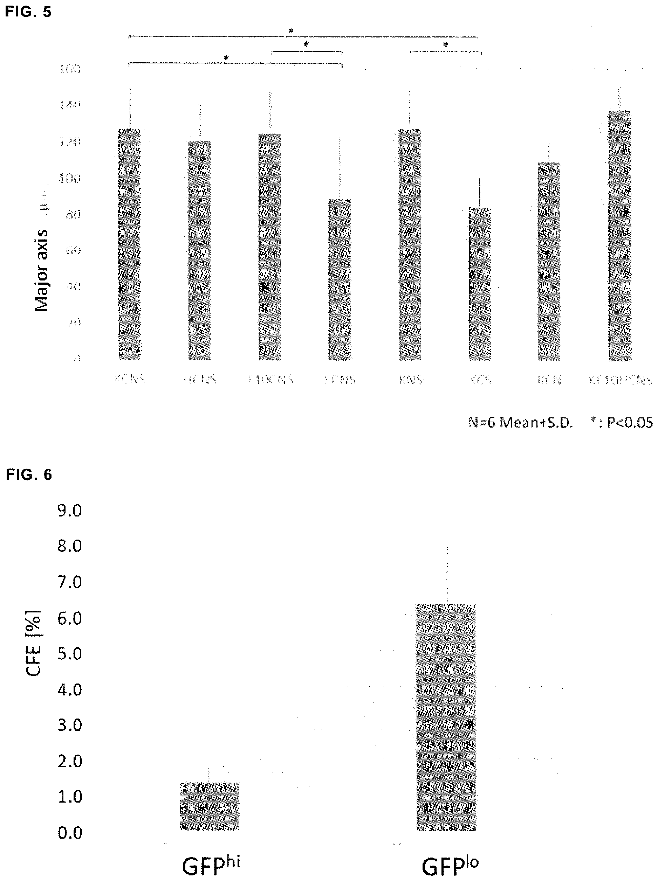

The GFP^{lo} suspension including lung epithelial stem cells was cultured in a manner similar to the above-described GFP^{hi} suspension including lung epithelial stem cells, and CFE [%] was calculated as in Test Example 1. Unlike the GFP^{hi} fraction, the culturing was carried out with 150 cells per well. The average and standard deviation of CFEs (n=6) were calculated (FIG. 4). The average and standard deviation of organoids' major axis (n=6) were further calculated (FIG. 5).

### - Combination of Four Addition Agents

Figures 4 and 5 show that combining four addition agents, "KCNS," resulted in a CFE of about 8.0% and a major axis of about 127 µm, respectively. The addition agents' combination "HCNS" or "F10CNS," in which the addition agent "H" or "F10" replaced the addition agent "K" in the "KCNS," was not significantly different in CFE or major axis from the "KCNS." The results suggest that the addition agent "K" in the four addition agents' combination "KCNS" is substitutable for "H" or "F10" regarding the efficient organoid formation. The addition agents' combination "ECNS," in which the addition agent "E" replaced "K" in "KCNS," was significantly lower in CFE than the "KCNS" (Tukey test, *: P<0.05).

### - Combination of Three Addition Agents

The GFP^{lo} fraction was also examined for the CFE and major axis obtained with combinations of the "KNS," "KCS," or "KCN," which removed the addition agent "C," "N," or "H" from the four addition agents' combination "KCNS," as in examined with the GFP^{hi} fraction. The "KCS" was significantly lower in CFE and major axis than the "KCNS" (Tukey test, *: P<0.05). The CFE obtained with the "KNS" was not significantly different from the CFE and major axis obtained with the "KCNS" but tended to be lower. The addition agents' combination "KNS," which removed the addition agent "C" from "KCNS," was not significantly different in CFE or major axis from the CFE of the "KCNS." This indicates that the result was the same as the GFP^{hi} fraction.

### - Combination of Six Addition Agents and Combination of Five Addition Agents

Figures 4 and 5 show that the six addition agents' combination "KF10HCNS" was not significantly different in the efficient organoid formation from the four addition agents' combination "KCNS" but tended to be higher. The "KF10HCNS" is not significantly different in the efficient organoid formation from the four addition agents' combinations "HCNS" and "F10CNS." The addition agent "C" in the six addition agents' combination "KF10HCNS" was not essential for the efficient organoid formation as described above. It is accordingly suggested that the five addition agents' combination "HF10KNS" is preferable for an efficient formation of organoids from the GFP^{lo} fraction.

### (GFP^{neg} Fraction)

CFEs [%] of the GFP^{neg} fraction suspension including lung epithelial stem cells were also calculated as with the GFP^{hi} fraction suspension including lung epithelial stem cells. Combining three addition agents, "KNS," resulted in a CFE of 0.75%.

Test Example 2 suggested that a combination consisting of at least one selected from the group consisting of addition agents, KGF (K), HGF (H), and FGF10 (F10); BMP inhibitor such as Noggin (N); and TGFβ inhibitor such as SB431542 (S) is preferable for efficient organoid formation. It suggested that a combination consisting of CHIR99021 (C), "N," "S," and "H" is further preferable, and a combination consisting of "K," "N," "S," "H," and "F10" or a combination consisting of "K," "C," "N," "S," "H," and "F10" is furthermore preferable for more efficient organoid formation. The addition of "C" is preferable for the efficient organoid formation of AT2, as demonstrated in Test Example 3.

### [Test Example 3]

Test Example 2 indicated that adding CHIR99021 (an agent capable of activating Wnt signaling) does not affect the efficiency of forming organoids. Test Example 3 examines the affection of CHIR99021 (C) in the differentiation of lung epithelial stem cells. A canonical Wnt inhibitor, XAV939 (X), was used. A suspension including lung epithelial stem cells from the GFP^{hi} and GFP^{lo} fractions was cultured as in Test Example 2 to form organoids. A four addition agents' combination "KCNS" or "KXNS" was added to the basal medium MTEC/B27 to produce a culture medium. From organoids formed after culturing, paraffin sections were prepared. The paraffin sections of the organoids were immunostained with an antibody against Sftpc and an antibody against Sox2, a marker of bronchial epithelium cells.

Regarding the GFP^{hi} fraction, Sox2 was weakly expressed in a portion of organoids formed with an addition agents' combination KCNS, which included CHIR99021 (C) being an agent capable of activating Wnt signaling. Sox2 was highly expressed in a portion of organoids formed with an addition agents' combination "KXNS," which included 10 µM of XAV939 (X) being a Wnt/p-catenin inhibitor. The addition agents' combination "KCNS" tends to express Sftpc highly compared to the addition agents' combination "KXNS." As demonstrated in Test Example 1, the GFP^{hi} fraction includes AT2 at a large portion and BASCs at a small portion. These results accordingly lead to a prediction that BASCs included in a part of the GFP^{hi} fraction differentiated to AT2 in the presence of the agent capable of activating Wnt signaling, resulting in the formation of organoids of AT2. It is predicted that organoids formed in the presence of the Wnt inhibitor include at least two types, organoids derived from the partially-included BASCs and organoids derived from the predominantly-included AT2.

Regarding the GFP^{lo} fraction, Sox2 was weakly expressed in the organoids using the addition agents' combination "KCNS," which included the agent capable of activating Wnt signaling. In contrast, Sox2 was highly expressed in the organoids using the addition agents' combination "KXNS," which included the Wnt inhibitor, the "KCNS" tends to express Sftpc highly compared to the "KXNS." The GFP^{lo} fraction results showed similar tendencies to those of the GFP"' fraction.

Test Example 3 suggests that forming organoids from lung epithelial stem cells in the presence of an agent capable of activating Wnt signaling (e.g., CHIR99021) results in the formation of AT2 organoids from cells whose type is different from AT2 cells.

### [Example 1] Forming organoids from lung epithelial stem cells

Lung epithelial stem cells in the GFP^{hi} fraction were seeded onto a 96-well ultra-low attachment plate (cell repellant 96-well plate) at 2,500 cells per well. They were cultured for 6 days with a culture medium to calculate CFEs (FIG. 6), as in Test Example 2. The used culture medium is the basal medium MTEC/B27, which was cooled on ice, supplemented with a combination of seven addition agents, Y-27632(Y), HGF(H), Fgf10(F10), KGF(K), CHIR99021(C), CHIR99021(N), and SB431542(S) and with 2.5% Matrigel. Like the above, lung epithelial stem cells of the GFP^{lo} fraction were seeded at 200 cells per well and cultured for 6 days, and then CFEs were calculated (FIG. 6).

Figure 6 shows that organoids of lung epithelial stem cells are formed by culturing the lung epithelial stem cells in a culture medium containing Matrigel at a low concentration of 2.5% and that the formation efficiencies of organoids were about 1.4% for the GFP^{hi} fraction and about 6.4% for the GFP^{lo} fraction. It is indicated that the results were comparable to those formation efficiencies of organoids from lung epithelial stem cells in a culture medium including a gel of 50% or 75% Matrigel were 0.86% for the GFP^{hi} fraction and 12% for the GFP^{lo} fraction.

Example 1 suggests that organoids of lung epithelial stem cells may be formed in a culture medium including a gel of extracellular matrix (e.g., 50% or 75% Matrigel) as a scaffold or in a culture medium containing extracellular matrix (e.g., 2.5% Matrigel) as a dispersed component as long as in the presence of the addition agents according to the present disclosure.

### [Test Example 4]

Whether the formed organoids possess a capacity to differentiate was examined. Lung epithelial stem cells from the GFP^{hi} fraction were cultured in a culture medium for 8 days, as in Test Example 2. The culture medium is the basal medium MTECIB27 supplemented with the four addition agents' combination "KCNS." One of two culture products was cultured in the culture medium for another 4 days, and the other was cultured in the basal medium for another 4 days. On day 12 after culturing, it was observed that the organoids formed by culturing in the basal medium highly expressed Hopx, a cell marker of AT1 cells that are differentiated pulmonary alveoli cells. Test Example 4 indicates that organoids formed in the presence of the addition agents according to the present disclosure possess a capacity to differentiate.

### [Preparation Example2]

### (Preparing suspensions including lung adenocarcinoma cells)

Prepared was Sftpc-CreERT2;KRASLSLG12D;Rosa26-mTmG mouse, which was generated by mating three types of mice, B6.129S-Sftpctm1 (crelERT2) Blh/J ("Sftpc-CreERT2") mouse (Jackson Lab, Stock No.028054), 86.129 (Cg)-Gt(ROSA) 26Sortm4 (ACTB-tdTomato,-EGFP) Luo/J (Rosa26-mTmG") mouse (Jackson Lab, Stock No.007676), and B6N.Cg-Krastm4 Tyj/CjDswJ (KrasLSLG12D) mouse (Jackson Lab, Stock No. 019104). In the Sfitpc-CreERT2;KRASL5LG12D;Rosa26-mTmG mice, the cell membrane of Kras-activated cell emits green fluorescence derived from EGFP.

The Cre recombinase is activated in Sftpc-CreERT2 without administration of tamoxifen, and recombination occurs in about 5-10% of cells (Barkauskas CE.et al., J Clin Invest. 2013;123(7):3025-36). Many nodules were observed in the lungs of Sftpc-CreERT2;KRASLSLG12D;Rosa26-mTmG mice, suggesting that cancer cells exist in the lungs of these mice.

Suspensions including lung epithelial cells were prepared with Sftpc-CreERT2;KRASLSLG12D;Rosa26-mTmG mice as in Preparation Example 1. Suspensions including Kras-activated lung adenocarcinoma cells were from the prepared suspensions including lung epithelial cells based on fluorescence from fluorophor-labeled anti-EpCAM antibody and fluorescence from GFP as in Preparation Example 1.

### [Example 2] Forming organoids from lung adenocarcinoma cells

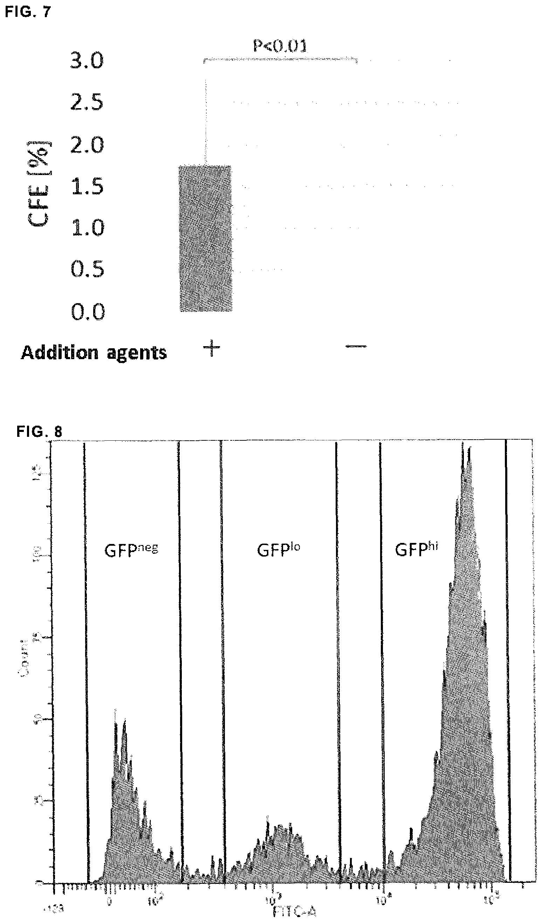

Lung adenocarcinoma cells obtained above were seeded onto a 96-well ultra-low attachment plate (cell repellant 96-well plate) at 2,500 cells per well. They were cultured for 6 days with a culture medium to calculate CFEs (FIG. 7), as in Test Example 2. The used culture medium is the basal mediumMTECIB27, which was cooled on ice, supplemented with the combination of seven addition agents, Y-27632(Y), HGF(H), Fgf10(F10), KGF(K), CHIR99021(C), CHIR99021(N), and SB431542(S) and with 2.5 % Matrigel. Like the above, the above-obtained lung adenocarcinoma cells were cultured in the culture medium, which was the basal mediumMTECIB27 supplemented with 2.5% Matrigel, for 6 days, and then CFEs were calculated (FIG. 7).

Figure 7 shows that culturing lung adenocarcinoma cells in the presence of the addition agents ("+" in Fig. 7) resulted in forming organoids and a CFE of about 1.7%. No organoid formation was observed from lung adenocarcinoma cells in the absence of the addition agents ("-" in Fig. 7).

Example 2 suggests that organoids of lung adenocarcinoma cells can be formed even in a culture medium that does not include a scaffold as long as it includes the addition agents according to the present disclosure.

### [Preparation Example3]

Suspensions including lung epithelial stem cells were obtained from the lungs of SFTPC-GFP mice and were separated into three pre-determined fractions (GFP^{hi}, GFP^{lo}, and GFP^{neg}) based on GFP-derived fluorescence intensities measured with FACS Aria II (BD) (FIG. 8) as in Preparation Example 1. The GFP^{hi} fraction corresponds to the P19 fraction in Test Example 2. The GFP^{lo} fraction corresponds to the P18 fraction in Test Example 2. The GFP^{neg} fraction corresponds to the P17 fraction in Test Example 2.

### [Test Example 5]

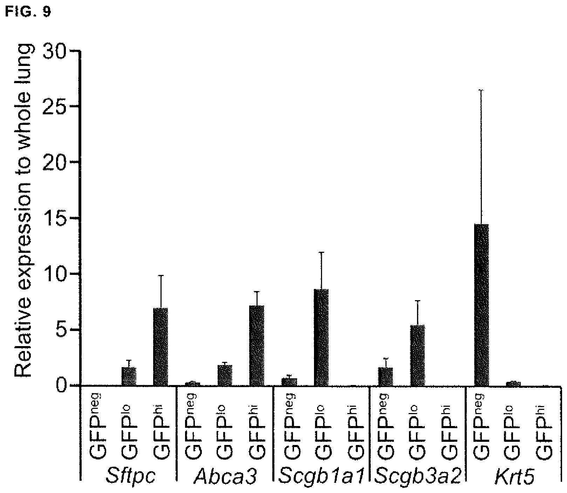

Marker genes were amplified by qPCR to examine the marker genes' expression patterns, and the cell types of cells included in each fraction were determined from the expression patterns as in Test Example 1 (FIG. 9). The result showed that the GFP^{neg} fraction predominantly included basal cells that Krt5 was positive and club cells that Scgb1a1 and Scgb3a2 were positive. The GFP^{neg} fraction also included Ciliated cells and AT1 cells like the P17 fraction in Test Example 1. It showed that the GFP^{lo} fraction predominantly included club cells that Scgb1a1 and Scgb3a2 were positive. It showed that the GFP^{hi} fraction predominantly included AT2 cells that Sftpc and Abca3 were positive and suggested including some BASCs.

### [Test Example 6]

Cells from each of the GFP^{hi}, GFP^{lo}, and GFP^{neg} fractions were cultured without feeder cells in a growth medium (including 50% Matrigel), which was the basal medium MTECIB27 supplemented with HGF (H), FGF10 (F10), KGF (K), NOGGIN (N), SB431542 (S), and CHIR99021 (C) at the final concentrations described in Table 2. CHIR99021(C) was added to the basal medium because CHIR99021(C) may be involved in the differentiation of club cells into AT2 cells though it is not essential for forming organoids. On day 9 after culturing, the growth medium was exchanged with the basal medium MTEC/B27, and the cells were cultured for another 3 days to form organoids in which their differentiation is accelerated.

Marker proteins' expression in the formed organoids before and after the acceleration of differentiation was examined by immunostaining with the following reagents.

**TABLE 6]**

| Name | Catalog number | Distributor | Remarks |
|---|---|---|---|
| CC10(B6) | sc-390313 | Santa Cruz | Host: mouse 1:500-1000 |
| Prosurfactant Protein C (proSP-C) | AB3786 | Millipore | Host: rabbit 1:300 |
| Human/Mouse/Rat SOX2 Antibody | 14-9965-82 | R&D | Host: goat 1:200 |
| Cytokeratin5 antibody (EP1601Y) (rabbit) | ab52635 | Abeam | Host: rabbit 1:500 |
| Monoclonal Anti-Acetylated Tubulin antibody produced in mice | T7541 | Sigma | Host: mouse 1:1000 |
| Anti-m/rRAGE purified Rat monoclonal IgG clone175410 | MAB1179 | R&D | Host: rat1:200 |
| Anti-HOPX antibody produced in rabbit | HPA030180 | Sigma | Host: rabbit1 :200 |
| Anti HT2-280 | TB-27AHT2-280 | TERRACE BIOTECH | Host: mouse 1:50 |
| Trypsin, for cell biology study derived from pig pancreas | T4799-5G | Sigma | |
| Antigen Unmasking solution | H-3300 | Vector | |
| Antigen Unmasking solution (high pH) | H-3301 | Vector | |
| M.O.M. (mouse on mouse) immunodetection kit | BMK-2202 | Vector | |

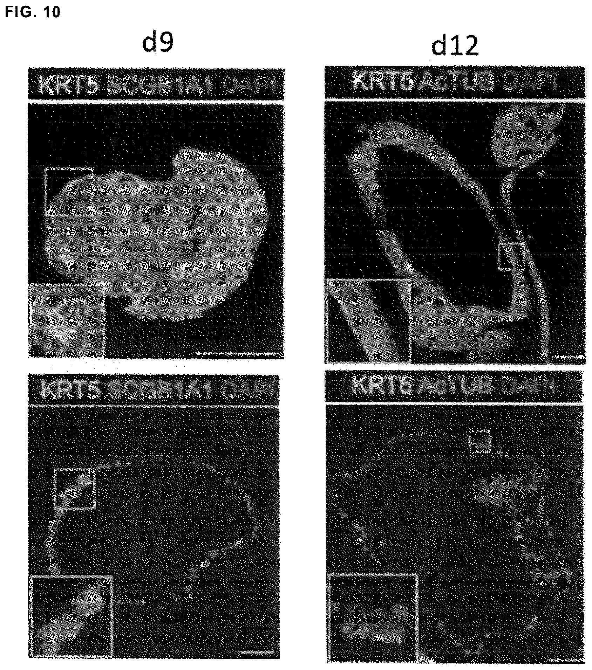

Culturing cells of the GFP^{neg} fraction led to forming of at least two types of organoids (FIG. 10). One type of organoids before the differentiation (d9) was composed of a cell population including basal cells that Krt5 (basal cell marker) was positive and club cells that SCGB1A1 (club cell marker) was positive. The differentiation conditions in Test Example 6 did not differentiate the cells constituting the organoids into Ciliated cells. The other type of organoids was composed, before the differentiation (d9), of a cell population including club cells that SCGB1A1 (club cell marker) was positive and composed, after the differentiation (d12), of a cell population including club cells that ACTUB (Ciliated cell marker) had become positive.

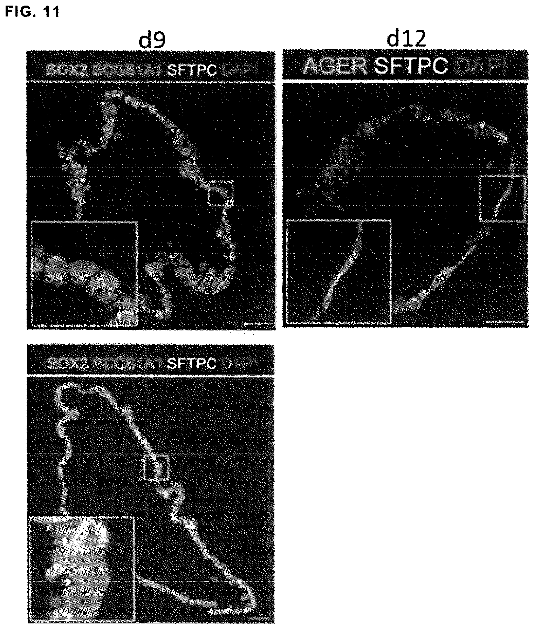

Culturing cells of the GFF^{lo} fraction led to forming of at least two types of organoids (FIG. 11). One type of organoids was composed, before the differentiation (d9), of a cell population including club cells that SCGB1A1 (club cell marker) was positive, cells that SOX2 (bronchial epithelium marker) was positive, and cells that SFTPC (AT2 cell marker) was positive and composed, after the differentiation (d12), of a cell population including pulmonary alveoli-lineage cells that AGER (AT1 cell marker) had become positive. The other type of organoids was composed, before the differentiation (d9), of a population of cells that SOX2 (bronchial epithelium marker) and SFTPC (AT2 cell marker) were positive and composed, after the differentiation (d12), of a cell population including pulmonary alveoli-lineage cells that AGER (AT1 cell marker) had become positive.

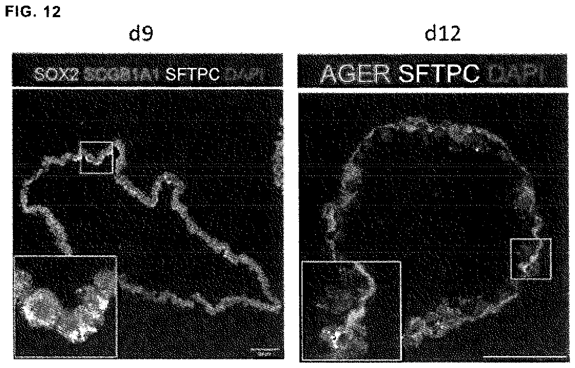

Culturing cells of the GFP^{hi} fraction led to forming of at least one type of organoid (FIG. 12). The organoids were composed, before the differentiation (d9), of a cell population including cells that SFTPC (AT2 cell marker) was positive and composed, after the differentiation (d12), of a cell population including AT2 cells that AGER (AT1 cell marker) was positive.

### [Test Example 7]

Whether combinations of addition agents described below are efficient in forming organoids is examined by using the GFP^{hi}, GFP^{lo}, and GFP^{neg} fractions' cells derived from SFTPC-GFP mice, as target cells. Gels containing the cells were formed with 50% or 75% Matrigel^{®} and cultured as in Test Example 2. The efficiencies in forming organoids were estimated from CFEs [%] and the major axis of organoids. Test Example 7 used six SFTPC-GFP mice and used, in total, 120,000 cells of the GFP^{hi} fraction, 3,600 cells of the GFP^{lo} fraction, and 24,000 cells of the GFP^{neg} fractions, respectively (n=6).

### (GFP^{hi} Fraction)

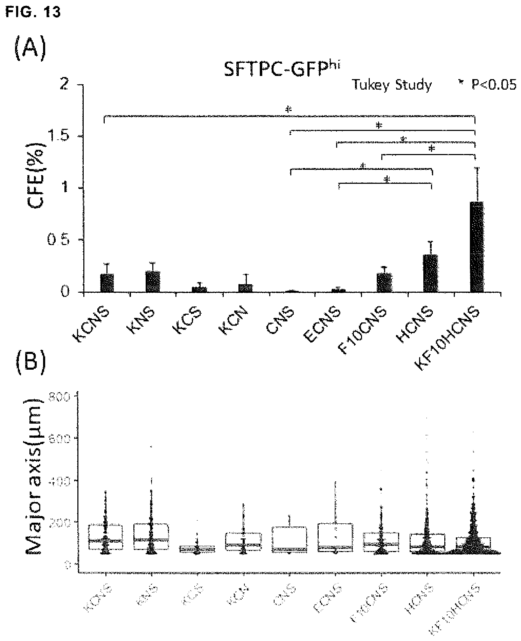

Lung epithelial stem cells from the GFP^{hi} fraction were cultured in a culture medium supplemented with an addition agents' combination described below to form organoids as in Test Example 2. The culturing was performed with 5,000 cells seeded onto a well. CFEs [%] and major axis [µm] were measured as in Test Example 2 (FIG. 13).

**[TABLE 7**

| | Name | **KCNS** | **KNS** | **KCS** | **KCN** | **CNS** | **ECNS** | **F10CNS** | **HCNS** | **KF10 HCNS** |
|---|---|---|---|---|---|---|---|---|---|---|
| **K** | KGF | + | + | + | + | - | - | - | - | + |
| **C** | CHIR99021 | + | - | + | + | + | + | + | + | + |
| **N** | Noggin | + | + | - | + | + | + | + | + | + |
| **S** | SB431542 | + | + | + | - | + | + | + | + | + |
| **H** | HGF | - | - | - | - | - | - | - | + | + |
| **F10** | FGF10 | - | - | - | - | - | - | + | - | + |
| **E** | EGF | - | - | - | - | - | + | - | - | - |

| | | | | | | | | | | |
|---|---|---|---|---|---|---|---|---|---|---|
| +: Added as an addition agent -: Not added as an addition agent | | | | | | | | | | |

Compared to the three addition agents' combination "CNS," the "KCNS," "KNS," "F10CNS," "HCNS," or "KF10HCNS" indicated a higher CFE value (FIG. 13A). Any of these addition agents' combinations were not significantly different in the major axis (FIG. 13B).

### - Combination of Six Addition Agents

Using the six addition agents' combination "KF10HCNS" resulted in the highest CFE, similar to the results in Test Example 2 (FIG. 13A). For example, the "KF10HCNS" was significantly higher in CFE than the four addition agents' combination "KCNS," "ECNS," or "F10CNS," which further combined the three addition agents' combination "CNS" with one receptor tyrosine kinase activator.

### - Combination of Four Addition Agents

The four addition agents' combination "HCNS" was significantly larger in CFE than the "CNS" or "ECNS" (FIG. 13A). The four addition agents' combinations, "KCNS," "F10CNS," and "HCNS," each provided comparable CFE (FIG. 13A). The results indicate that the addition agents "K," "F10," and "H" are substitutable regarding the efficient organoid formation, similar to the results in Test Example 2.

### - Combination of Three Addition Agents

The three addition agents' combination "KCS" that removed the addition agent "N" from the four addition agents' combination "KCNS" or the three addition agents' combination "KCN" that removed the addition agent S, each tended to be lower in CFE than the "KCNS" (FIG. 13A). The results indicate that the addition agents "N" and "S" are important in the four addition agents "KCNS" to render the organoid formation efficiently, similar to the results in Test Example 2.

The addition agents' combination "KNS," which removed the addition agent C from the KCNS, was not significantly different in CFE from the "KCNS" (FIG. 13A). The result indicates that the addition agent "C" is not essential for the efficient organoid formation, similar to the results in Test Example 2. On the other hand, the addition agent "C" is indicated to induce the differentiation of lung epithelial stem cells, which constitute cell aggregates, as demonstrated in Test Example 3.

### (GFP^{lo} Fraction)

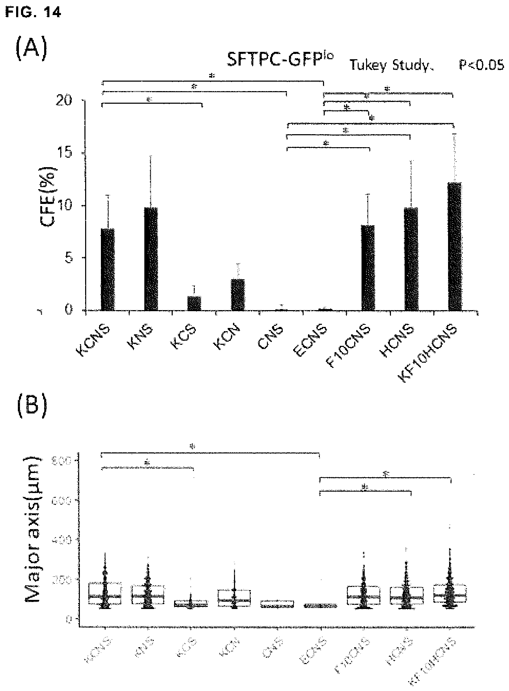

CFEs [%] of the lung epithelial stem cells from the GFP^{lo} fraction were calculated as described in the GFP^{hi} fraction. Note that 150 cells were cultured per well, unlike the GFP^{hi} fraction. The mean and standard deviation of CFEs (n=6) were calculated (FIG. 14A). The mean and standard deviation of organoids' major axis (n=6) were also calculated (FIG. 14B).

Compared to the three addition agents' combination "CNS," the "KCNS," "KNS," F10CNS," "HCNS," or "KF10HCNS" indicated a higher CFE value (FIG. 14A).

### - Combination of Six Addition Agents

Using the six addition agents' combination "KF10HCNS" resulted in the largest CFE and major axis (FIGs. 14A and B), similar to the results in Test Example 2. For example, the "KF10HCNS" was significantly higher in CFE than the three addition agents' combination "CNS" or the four addition agents' combination "ECNS," which further combined the three addition agents' combination with EGF(E) (FIG. 14A). The "KF10HCNS" was significantly longer in the major axis than the "ECNS" (FIG. 14B).

### - Combination of Four Addition Agents

The four addition agents' combination "F10CNS" or "HCNS" was significantly higher in CFE than the three addition agents' combination "CNS" (FIG. 14A). The four addition agents' combinations "KCNS," "F10CNS," and "HCNS," respectively, indicated comparable CFEs (FIG. 14A). The results indicated that the addition agents "K," "F10", and "H" are substitutable regarding the efficient organoid formation, similar to the result of Test Example 2. The four addition agents' combination "ECNS" that the addition agent "E" replaced the addition agent "K" in the "KCNS" was significantly lower in CFE and major axis than the KCNS (FIGs. 14A and B). These results indicate that the addition agent "E" cannot be substituted for addition agents "K," "F10," or "H" regarding the efficient organoid formation.

### - Combination of Three Addition Agents

The three addition agents' combination "KCS" or "CNS," removed the addition agent "N" or "K" from the four addition agents' combination "KCNS," respectively, was significantly lower in CFE than the "KCNS" (FIG. 14A). The use of the "KCNS" resulted in a significantly shorter major axis than the "KCS" (FIG. 14B). The three addition agents' combination "KCN," which removed the addition agent S from the "KCNS," tended to be lower in CFE than the "KCNS (FIG. 14A). These results indicate that the addition agents "N" and S" are important for the efficient organoid formation, similar to the results in Test Example 2.

The addition agents' combination "KNS," which removed the addition agent "C" from the "KCNS," was not significantly different in CFE from the "KCNS" (FIG. 14A). The result indicates that the addition agent "C" is not essential for the efficient organoid formation, similar to the results in Test Example 2.

### (GFP^{neg} Fraction)

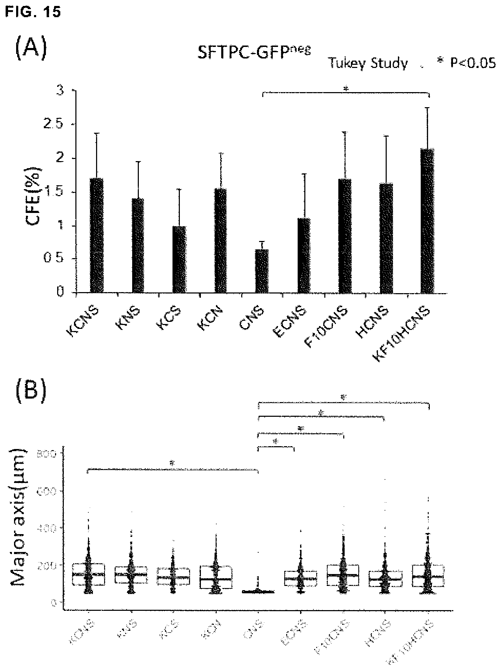

CFEs [%] of the lung epithelial stem cells from the GFP^{neg} fraction were measured as described in the GFP^{hi} fraction. Note that 1,000 cells were cultured per well, unlike the GFP^{hi} fraction. The mean and standard deviation of CFEs (n=6) were calculated (FIG. 15A). The mean and standard deviation of organoids' major axis (n=6) were also calculated (FIG. 15B). The "KCNS," "KNS," F10CNS," "HCNS," or "KF10HCNS" indicated a higher CFE value compared to the three addition agents' combination "CNS" (FIG. 15A). These combinations of addition agents include "N" and "C," and also include at least one selected from the group consisting of "K," "H," and "F10."

### - Combination of Six Addition Agents

Using the six addition agents' combination "KF10HCNS" resulted in the largest CFE and major axis (FIGs. 15A and B). For example, the "KF10HCNS" was significantly higher in CFE than the three addition agents' combination "CNS," which did not include receptor tyrosine kinase activator (FIG. 15A).

The use of the four addition agents' combination "KCNS," "ECNS," "F10CNS," or "HCNS," combined the three addition agents' combination "CNS" with one receptor tyrosine kinase activator, resulted in a significantly larger major axis than the three addition agents' combination "CNS" (FIG. 15B). The results indicate that combining the three addition agents' combination "CNS" with any one of the four receptor tyrosine kinase activators "K," "E," "F10," and "H" is important to form organoids.

Using the ECNS for cells of the GFP^{neg} fraction was significantly different in organoid formation compared to the use of CNS, unlike cells of the GFP^{hi/lo} fraction (FIG. 13A, FIG. 14A, and FIG. 15B). The result corresponds to the use of EGF in culturing basal cells without using Feeder cells (Proc Natl Acad Sci USA. 2009 Aug 4;106(31):12771-5).

Test Example 7 suggested that a combination consisting of at least one selected from the group consisting of addition agents, KGF (K), HGF (H), and FGF10 (F10); BMP inhibitor such as Noggin (N); and TGFβ inhibitor such as SB431542 (S) is preferable for the efficient organoid formation. It suggested that a combination consisting of CHiR99021 (C), "N," "S," and "H" is further preferable, and a combination consisting of "K," "N," "S," "H," and "F10" or a combination consisting of "K," "C," "N," "S," "H," and "F10" is furthermore preferable for more efficient organoid formation.

### [Example 3] Transplantation of organoids derived from club cells into lung-injured mice

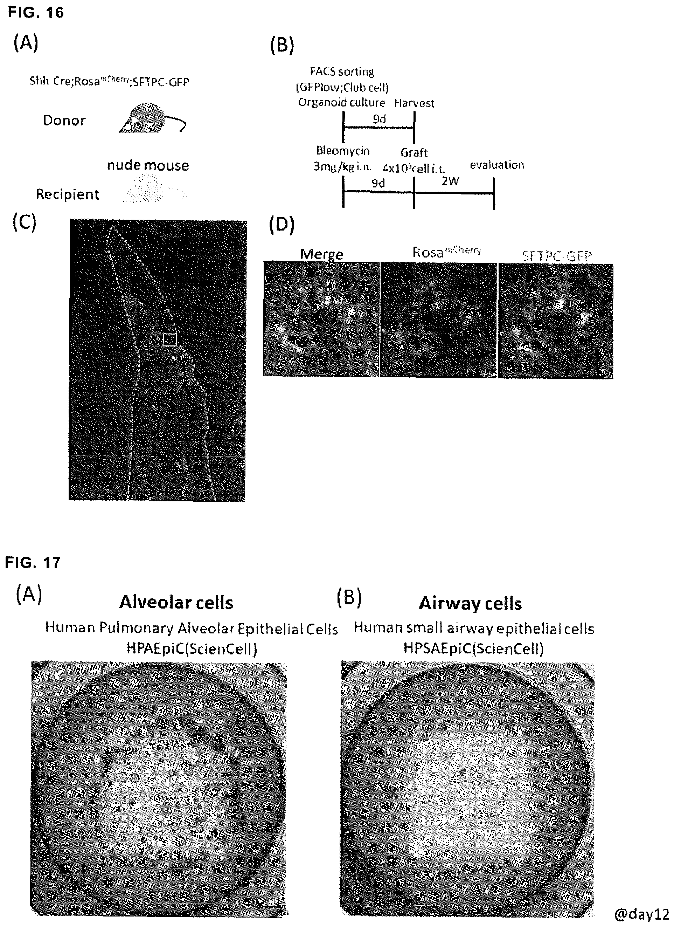

Genetically modified rShh-Cre;Rosa26-CAG-LSL-H2B-mCherry;SFTPC-GFP mice were prepared (FIG. 16A, donor mice). The genetically modified mouse was generated by mating three types of mice, B6.Cg-Shhtm1 (EGFP/cre)Cjt/J mouse (abbreviated herein as "Shh-Cre") (Jackson Lab. Stock No.005602), B6;129S-Gt(ROSA)26Sortm1.1Ksvo/J mouse (abbreviated herein as "ROSA26-mCherry") (Jackson Lab. Stock No.023139), and SFTPC-GFP mouse.

A population of cells (club cells) from the GFP^{lo} fraction was obtained from the genetically modified mice as in Preparation Example 1. The obtained cellular nucleus was stained with mCherry. GFP was highly expressed when the cells became AT2 cells.

According to the methods below, organoids were formed from the cells obtained from the genetically modified mice, and the organoid-derived cells were administered to lung-injured mice (FIG. 16B). The cell population including the cells, obtained from the genetically modified mice, in the medium (MTECIB27+YHF10KCNS+2.5% Matrigel 2mL) was seeded onto a 6-well plate at 1×10⁴ cells per well. A matrigel-free medium of 1 mL was added to the wells once every 3 days and cultured for 9 days in total. On day 9 after culturing, the culture medium including organoids was centrifuged (400 G, 3 min, 4°C) to collect the organoids. The collected organoids were mixed with 10 mL of protease solution (Accutase, Collagenase type I (450U/mL, Worthington), DNase (0.1 mg/mL, SIGMA)) pre-warmed at 37°C, and were gently stirred with a rotator at 37°C for 20 minutes. The above solution was pipetted to obtain a cell suspension, and the suspension was centrifugated (400 G, 5 min, 4°C) to collect cell aggregates. The collected cell aggregates were mixed with 5 mL of 0.25% trypsin (Gibco, containing 0.1mg/mL of DNase) and were gently stirred with a rotator at 37°C for 10 minutes. The solution was pipetted to obtain a cell suspension, and the cell suspension was mixed with 10 mL of DMEM/F12 (containing 10% FBS) to stop the protease reaction. The cell suspension was centrifuged (400G, 5 min, 4°C), and the collected cells were suspended in 90 µL of the medium (MTEC/B27+"YHF10KNS") such that the medium of 75 µL includes 4×10⁵ viable cells.

Lung-injured mice were prepared, before 9 days to inject cells derived from the organoids, by anesthetizing nude mice with isoflurane (Pfizer) and intranasally administering bleomycin solution (Nippon Kayaku) at 3 mg/Kg volume.

The lung-injured mice were anesthetized with isoflurane (Pfizer) on the day to inject the organoid-derived cells, and the cell suspension prepared above was injected into the mice through the trachea. The lung-injured mice were dissected after two weeks of injecting the cells, and frozen sections of the lungs were prepared. Images of mCherry and GFP-derived fluorescence in the frozen sections were taken with fluorescence microscopy and examined for adhering the administered cells to and surviving in the lung tissue (FIGs. 16C and D).

A lung section shows cells emitted mCherry-derived fluorescence at the cellular nucleus (FIG. 16C). The result indicates that the club cells, obtained after the collection of club cells from the genetically modified mice as being a donor and the formation of organoids from the club cells, adhered to and survived in the lung tissue of the lung-injured mouse being a recipient. Among cells emitting mCherry-derived fluorescence at the cellular nucleus, some cells emitted GFP-derived fluorescence (FIG. 16D). The result indicates that AT2 cells, differentiated from club cells, adhered to and survived in the lung tissue of the lung-injured mice.

### [Example 4] Organoid formation from primary human cells

Purchased were the human alveolar cells (Human Pulmonary Alveolar Epithelial Cells: HPAEpiC) and human airway cells (Human Small Airway Epithelial Cells: HPSAEpiC}, which were described below.

**[TABLE 81**

| Name | Catalog number | Distributor | Lot number |
|---|---|---|---|
| Human Small Airway Epithelial Cells(HPSAEpiC) | 3230 | ScienCell | 28775 |
| Human Pulmonary Alveolar Epithelial Cells(HPAEpiC) | 3200 | ScienCell | 28699 |

The purchased frozen primary human cells were thawed at 37°C, and 100 µl of the thawed solution was suspended in 2 mL of the culture medium (MTEC/B27+"YHF10KCNS"+5% Matrigel). The cell suspension was seeded onto a 6-well repellent plate such that the seeded HPAEpiC was 1.44 × 10⁵ cells per well and the added HPSAEpiC was 5.6 × 10⁴ cells per well. On day 3 after culturing, the culture medium of 1 ml was added. The cultured cells were passed on day 6 after culturing such that the cells were at 2 × 10⁵ cells per well. After that, the cells were passed on every 12 days.

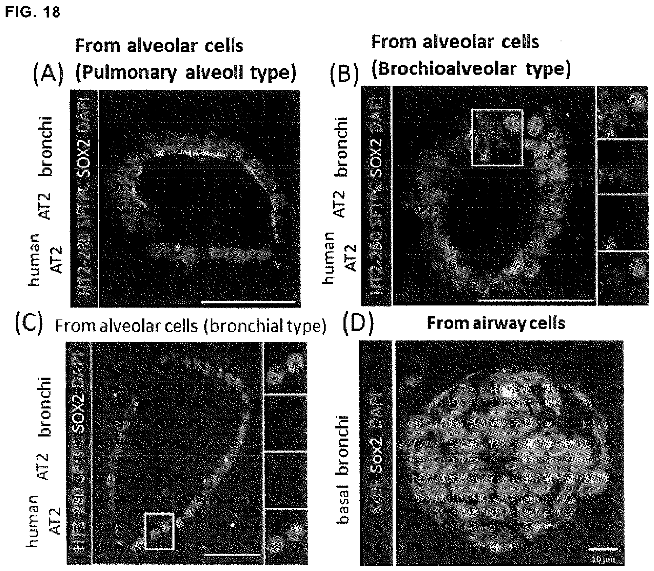

The cells passed on day 6 after culturing the human primary cells were cultured and observed on day 12 after culturing (Fig. 17). Organoids were observed in wells where human alveolar cells and human airway cells were cultured, respectively (FIGs. 17A and B). The reagents listed in Table 4 of Test Example 6 were used for the immunostaining to investigate the expression of marker proteins in the formed organoids (FIG. 18). The Immunostaining was performed on paraffin sections of the organoids. The results indicated that three types of organoids were formed from the human alveolar cells (FIGs. 18A-C), and one type of organoids were formed from the human airway cells (FIG. 18D).

Figure 18A shows that the organoid formed from the human alveolar cells is a pulmonary alveoli type that expresses a human-specific AT2 cell marker (HT2-280) and an AT2 cell marker (SFTPC) common to mice. Figure 18B shows that the organoid formed from the human alveolar cells is a bronchoalveolar type that expresses the two marker proteins, HT2-280 and SFTPC, and a bronchial epithelium marker SOX2. Figure 18C shows that the organoid formed from the human alveolar cells is a bronchial type expressing only a bronchial epithelium marker, SOX2. Example 4 demonstrated that three types of organoids were formed from human alveolar cells. The results suggest a reason that the purchased human alveolar cells were contaminated by cells from the peripheral airways.

Figure 18D shows that the organoid formed from human airway cells is composed of basal cells expressing a basal cell marker (KRT5) and a bronchial epithelium marker (SOX2).

Example 4 shows that culturing primary human lung cells in a culture medium containing a combination of the addition agents disclosed herein can lead to forming organoids.

### [Example 5] Imaging of organoid formation by culturing a cell population

Lung epithelial cells were prepared from genetically modified mice, Scgb1a1-CreER;Rosa26-mTmG. Administering Tamoxifen to the genetically modified mouse leads to incorporating Scgb1a1 into Rosa26 locus by a Cre recombinase. As a result, expression of Scgb1a1 can be detected by fluorescence from cell membrane-localized GFP (mGFP).

Tamoxifen was administered to the genetically modified mice five times. Three weeks after the administration of tamoxifen, the trachea was resected to remove the lungs. A cell suspension was prepared from the removed lungs as in Preparation Example 1. The cell suspension was mixed with the following antibodies and allowed to react on ice for 20 minutes: anti-EPCAM-PE-Cy7 (#25-5791-80, eBioscience), anti-CD24-APC (#25-0242-80, Invitrogen), anti-CD45-biotin (#13-0451, eBioscience), anti-CD31-biotin (#13-0311, eBioscience). The reaction solution was suspended in DPBS(-)/3% FBS of 500 µL and was centrifuged (400g, 3 min, 4°C). The precipitated cells were resuspended in DPBS(-)/3% FBS of 100 µL. The cell suspension was mixed with streptavidin APC-Cy7 of 0.25 µL per 1 × 10⁶ cells and allowed to react on ice for 10 minutes. The cells were suspended in DPBS(-)/3% FBS of 500 µL, centrifuged (400g, 3 min, 4°C), and the precipitated cells were resuspended in DPBS(-)/3% FBS of 500 µL. The cell suspension was mixed with 7-Amino-Actinomycin D (7-AAD) of 5 µL per 1 × 10⁶ cells and allowed to react on ice for 20 minutes. From the reaction solution, cell aggregates were taken away with a 40 µm Cell Strainer, and subjected to FACS.

FACS was performed to remove dead cells stained with 7-AAD, endothelial cells stained with anti-CD31, and hematopoietic cells stained with anti-CD45 from the prepared cell suspension. From the remaining cell population, epithelial cells stained with anti-EPCAM antibodies were collected, Scgb1a1-positive cells (mainly club cells) emitting GFP-derived fluorescence were collected, and tissue stem cells/progenitor cells stained with anti-CD24 antibody (CD24^{lo}) were collected. The collected cells were centrifuged (400g, 5 min, 4°C), and the precipitated cells were suspended in the culture medium (MTEC/B27+5% FBS+"YHF10KNS"), with which the culture medium (MTEC/B27+5% FBS+"YHF10KNS") was mixed to prepare a cell suspension including 300 cells per 250 µL. The cell suspension was seeded onto 96-well ultra-low attachment plates (CELLSTAR, #655970, Greiner), and the 96-well plates were placed in a microscope integrated with an incubator, Celldiscoverer7 microscope (Zeiss). While the cells were incubated for 10 days, the cultured cells were imaged in a bright field at predetermined intervals. The imaging was conducted every 4 hours on the first day (day 0) and once a day from the 1st day to the 10th day. An objective lens used was with a magnification of 10x. Seventeen images were taken at an interval of 45µm in the Z-axis. Accordingly, the taken image file contains data including 17 images in the Z-axis direction at each time.

### (Method for analyzing Images)

A software for analyzing images, ZEN3.0 (blue edition), was used to convert the taken image files to Tiff files, and then a software for analyzing images, image J (ver. 1.52n), was used to analyze them. The process of forming an organoid from a single cell was traced through the image files from day 0 to the 10th day. When a cell aggregate grew in the major axis of not less than 50 µm, it was determined as the formation of an organoid. The analysis in tracing the organoid formation excluded cells out of focus, cells whose entire feature could not be caught due to the location at the edge in the tiling processing, cells contacted with another cell, and cells that could not be traced by day 6 after culturing for some reason (e.g., cells overlapped with grown organoids). Image files were created by cropping the regions where the formation process of an organoid from a single cell in the image data of day 0 could be observed. A plugin for image J, StackReg (http://bigwww.epfl.ch/thevenaz/stackreg/), was used to integrate the cropped image files having image data in the Z-axis direction. Three-dimensional constructed cells in the integrated image data were measured for cell morphology. The morphology measurements included area, perimeter, major axis, minor axis, circularity, gray value, and centroid.

### (Results)

The formation processes of organoids were observed for individual cells in a cell population of 1,056 cells, which were from a cell suspension obtained from three mice and cultured. Of the 1,056 cells, 235 cells formed organoids, and the other 821 cells did not form organoids. The organoid-forming and non-organoid-forming cells were measured for cell morphology, respectively (Fig. 19). Figure 19A shows that the organoid-forming cells were significantly larger in area than the non-organoid-forming cells. Figures 19B-19D show that the organoid-forming cells were significantly larger in perimeter, major axis, and minor axis, respectively than the non-organoid-forming cells. Figures 19E-19H show no significant differences in circularity, gray value (average, mode), and centroid between the organoid-forming and non-organoid-forming cells.

Example 5 indicates that, among lung epithelial cells obtained from the mice, relatively large cells, particularly cells whose area, perimeter, major axis, and/or minor axis are large, are more likely to form organoids.

### [Example 6] Imaging of organoid formation by culturing single cells

Culturing a cell population and observing individual cells in the cell population through the course of the culturing process, as in Example 5, occasionally resulted in impossible to trace the organoid formation process due to an overlap of the cell with another cell over time, etc. Example 6 accurately investigated the morphological features of a cell that likely forms an organoid by seeding and culturing a single cell on one well.

A cell suspension including lung epithelial cells was prepared from mice and then subjected to FACS to collect club cells, as in Example 5. The club cells were suspended in the culture medium (MTEC/B27+5% FBS+"YHF10KNS") to prepare a cell suspension including 2,500 to 5,000 cells per ml of the culture medium. The cell suspension of 2 ml was seeded onto Elplasia 6-well plates (Corning 4444) which were rendered non-adherent with Biosurfine AWP-MRH (6% aq) (Toyo Gosei), and the plates were then placed for 30 minutes. Then, a cell picking & imaging system, Cell Handler^{®} (Yamaha), was used to take bright field (BF) images and propidium iodide (PI. #130-093-233, Miltenyi Biotec)-derived fluorescence images of the plates' wells and to pick up and transfer individual PI-negative, living cells to 50 µL of the culture medium (MTEC/B27+5% FBS+"YHF10KNS"+2.5% Matrigel) in each well of a 384-cell plate (CELLSTAR, #655892, Greiner). The individual cells in each well were incubated at 37°C under 5% CO₂ for 10 days to form organoids. When a cell aggregate grew in the major axis of not less than 50 µm, it was determined as the formation of an organoid. The formation processes of organoids from single cells were imaged at the same intervals as in Example 5. image J (ver. 1.52n) was used for the taken image data to measure items of cell morphology, as in Example 5.

### (Results)

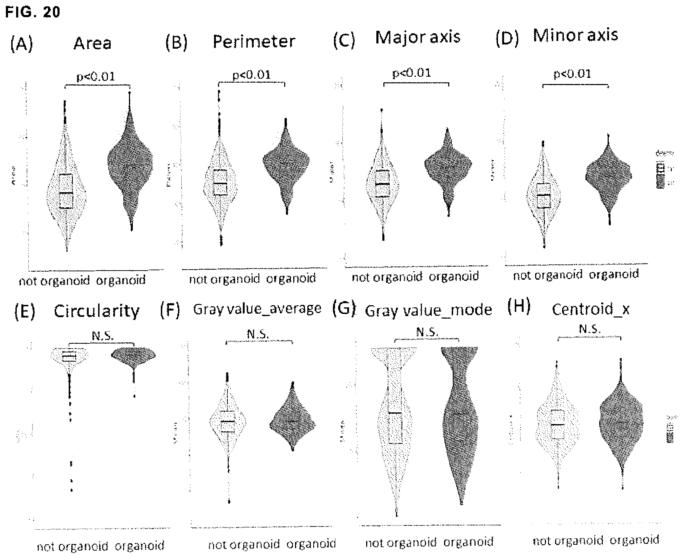

The formation processes of organoids from single cells were observed by culturing each of 1,053 cells, prepared from three mice, in individual wells. Of the 1,053 cells, 307 cells formed organoids, and the other 746 cells did not form organoids. The organoid-forming and non-organoid-forming cells were measured for cell morphology, respectively (FIG. 20). The organoid-forming cells were significantly larger in area, perimeter, major axis, and minor axis, respectively, than the non-organoid-forming cells (FIGs 20A to 20D). There were no significant differences in circularity, gray value (average, mode), and centroid between the organoid-forming and non-organoid-forming cells (FIGs. 20E to 20H).

Example 6 indicates that, among the lung epithelial cells obtained from the mice, relatively large cells, particularly cells whose area, perimeter, major axis, and/or minor axis are large, are more likely to form organoids, as in Example 5.

### [Example 7] Combination of measurement of cell morphometry and RNA sequencing in single cells

The cell morphometry data from single molecules are combined with RNA sequencing data from single molecules to examine gene expression patterns in cells possessing the morphological features revealed in Examples 5 and 6.

Club cells were collected from lung epithelial cells obtained from mice and suspended in the culture medium (MTEC/B27+5% FBS+"Y") to prepare a cell suspension including 2,500 to 5,000 cells per ml of the culture medium as in Example 6. The cell suspension of 2 ml was seeded onto Elplasia 6-well plates (Corning 4444) which were rendered non-adherent with Biosurfine AWP-MRH (6% aq) (Toyo Gosei), and the plates were placed for 30 minutes. Then, Cell Handler^{®} (Yamaha) was used to take BF images and PI images of the plates' wells and to pick up and transfer individual Pi-negative, living cells to 2 µL of RNase inhibitor solution (RNAsin plus 0.2 µL, 5x Maxima H-buffer 0.4 µL, RNase free water 1.4 µL, RNase free water 1.4 µL) in each well of a 96-well plate for PCR. RNA sequencing was performed on each of the cells to obtain RNA sequencing data of single cells. Further, Image J (ver. 1.52n) was used to measure the items of cell morphology from the image data of each cell, as in Example 5. An analyzing software, Seurat (ver. 3), was used to combine the image-analyzed data regarding the cell morphology measured with the RNA sequencing data from single cells.

### (Results)

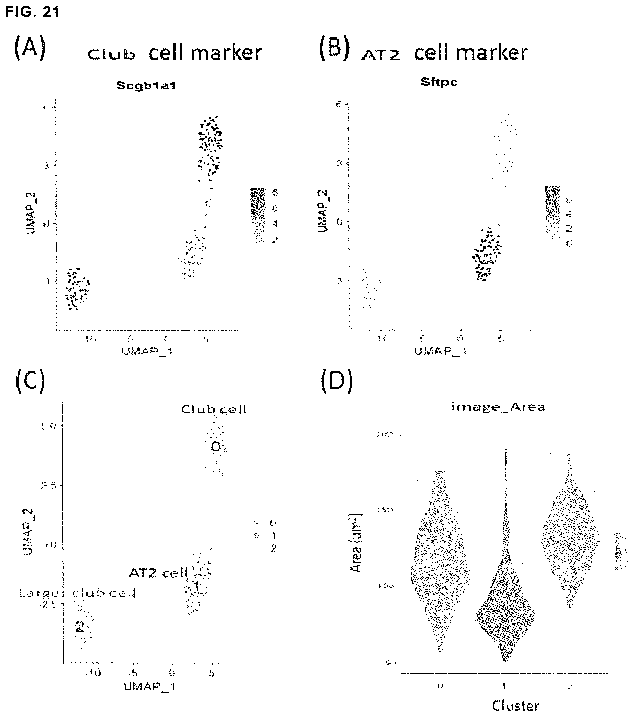

The image-analyzed data regarding the cell morphology from single cells were combined with the RNA sequencing data from single cells to conduct a clustering analysis, resulting in the measured cells being primarily classified into three clusters (FIG. 21). In Figures 21A-C, the cluster shown in the upper right is referred to as "Cluster 0," the cluster shown in the lower right is referred to as "Cluster 1," and the cluster shown in the lower left is referred to as "Cluster 2." Figure 21A shows the expression level of a club cell marker, Scgb1a1, in each cluster, indicating that Clusters 0 and 2 highly expressed Scgb1a1. The result indicates that the cells corresponding to Clusters 0 and 2 are club cells (FIG. 21C). Figure 21B shows the expression level of an AT2 cell marker, Sftpc, in each cluster, indicating that Cluster 1 highly expressed Sftpc. The result indicates that the cells corresponding to Cluster 1 are AT2 cells (FIG. 21C).

Figure 21D shows the area of cells corresponding to each Cluster. Figure 21D indicates that the club cells corresponding to Cluster 2 (right side in FIG. 21D) are larger in area than the club cells corresponding to Cluster 0 (left side in FIG. 21D). Considering the results in Examples 4 and 5, it is suggested that the relatively large club cells corresponding to Cluster 2 possess a higher tendency to form organoids.

Combining the image-analyzed data regarding the cell morphology with the RNA sequencing data from single cells revealed that the cells corresponding to each Cluster highly expressed specific genes. The table below shows the top 20 genes highly expressed in each Cluster.

**[TABLE 9]**

| Top ranking of expression level | Cluster 0 | Cluster 1 | Cluster 2 |
|---|---|---|---|
| 1 | Cbr2 | Lamp3 | Tff2 |
| 2 | Hp | Lyz2 | Reg3g |
| 3 | Cyp2f2 | Napsa | Bpifib1 |
| 4 | Prdx6 | Slc34a2 | Muc5b |
| 5 | Scgb1a1 | Lyz1 | Sult1d1 |
| 6 | Ldhb | Rnase4 | Fxyd 3 |
| 7 | Ces1d | Hc | Scgb3a1 |
| 8 | Cckar | Cd74 | Lypd2 |
| 9 | Selenbp1 | Scd1 | Chad |
| 10 | Gstm2 | Sftpc | S100a6 |
| 11 | Scnn1b | Lgi3 | Pglyrp1 |
| 12 | Scgb1c1 | Cldn18 | Aldh3a1 |
| 13 | Plpp3 | Etv5 | Bpifa1 |
| 14 | Ephx1 | Cxcl15 | Gsto1 |
| 15 | Dcxr | S100q | Lgals3 |
| 16 | Alas1 | Elovl1 | Pigr |
| 17 | Retnla | Fas | Scgb3a2 |
| 18 | Sec1413 | H2-Aa | Ltf |
| 19 | Ptgr1 | Fabp5 | Qsox1 |
| 20 | Krtap17-1 | Rn4.5s | Cyp2a5 |

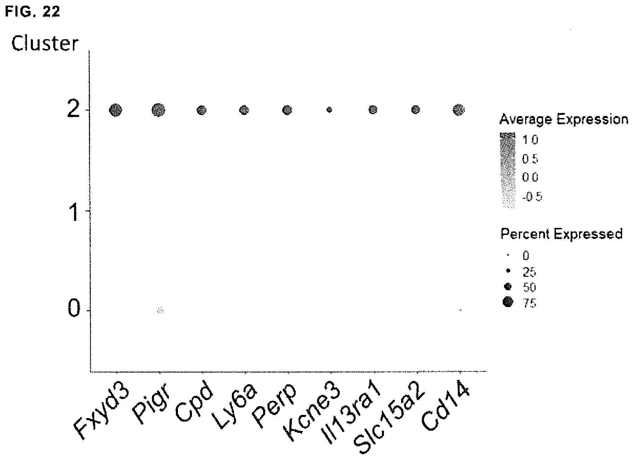

Examined were the transcriptional levels of genes corresponding to proteins highly expressed in cells of Cluster 2 compared to those in cells of the other Clusters (FIG. 22). The result revealed that Cluster 2 relatively highly expressed Fxyd3, Pigr, Cpd, Ly6a, Perp, Kcne3, Il13ra1, Sic15a2, and Cd14, relatively.

Examples 5-7 suggest that selecting lung epithelial cells expressing the markers revealed in Example 7 can select lung epithelial cells with a higher tendency to form organoids.

### [Example 8] Organoid formation from CD14⁺ club cells

CD14 among the markers revealed in Example 7 was used to isolate CD14⁺ cells expressing CD14 (cells corresponding to Cluster 2) from lung epithelial cells and to examine whether CD14⁺ cells were more likely to form organoids.

A cell suspension was prepared for subjecting to FACS as in Example 5, except that the following antibodies were used: anti-CD14-APC (#17-1401-81, Invitrogen), anti-MHC class2-eFluoro450 (#48-5321, eBioscience), anti-CD24-PEcy7 (#25-0242-80, Invitrogen), anti-CD45- biotin (#13-0451, eBioscience), and anti-CD31-biotin (#13-0311, eBioscience).

### (CD14⁺ cell fractionation)

FACS was performed to remove dead cells stained with 7-AAD, endothelial cells stained with the anti-CD31, hematopoietic cells stained with the anti-CD45 antibody, and AT2 cells stained with the anti-MHC class 2 antibody (cells corresponding to Cluster 1) from the prepared cell suspension. From a population of the remaining cells, Scgb1a1-positive cells (mainly club cells) emitting GFP-derived fluorescence were collected, and tissue stem cells/progenitor cells (CD24^{lo}) stained with the anti-CD24 antibody were collected. These resulted in fractionating the cells corresponding to Cluster 0 and Cluster 2. CD14⁺ cells stained with the anti-CD14 antibody and CD14⁻ cells not stained with the antibody were fractionated. The CD14⁺ and CD14⁻ cells were fractionated by FACS as follows: First, subjecting the cell suspension with isotype antibodies to FACS and setting a gate to cover the obtained cell distribution. Then, subjecting the cell suspension with the anti-CD14 antibody to FACS, fractionating the cells that exceeded the set gate area as CD14⁺ cells, and fractionating the cells within the set gate area as CD14⁻ cells.

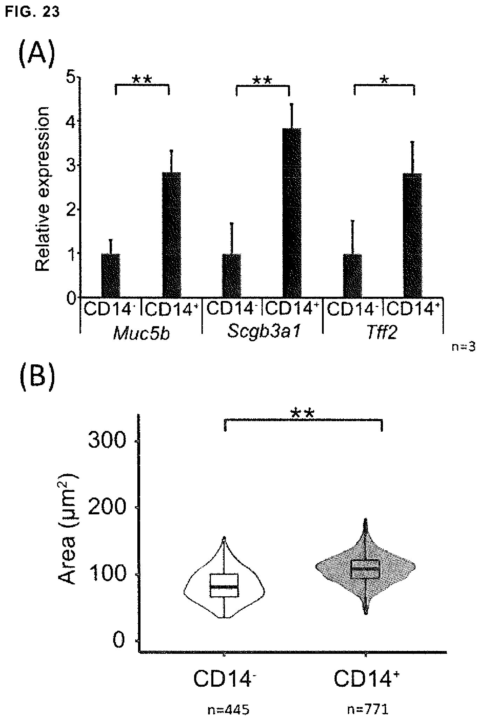

To examine whether the CD14⁺ cells are club cells corresponding to Cluster 2, the expression levels of Muc5b, Scgb3a1, and Tff2, which were genes found highly expressed in the cells of Cluster 2, were examined by qPCR (FIG. 23A). Figure 23A shows that the expression levels of Muc5b, Scgb3a1, and Tff2, marker genes, found highly expressed in the cells corresponding to Cluster 2, were high in the CD14⁺ cells. The result suggests that the CD14⁺ cells are club cells corresponding to Cluster 2.

It was further examined whether the CD14⁺ cells were relatively large and highly likely to form organoids (FIG. 23B). The obtained CD14^{+/-} cells were centrifuged (400G, 5 min, 4°C), resuspended in a basal medium of 250 µL, mixed with PI of 5 µL, dispersed to a black 96-well glass bottom plate (Sensoplate, Greiner#655892), and the 96-well plate was then placed for 30 minutes. Then, bright-field (BF) images and GFP/PI fluorescence images were taken for each well. Dead cells stained with PI were excluded from subsequent analysis. The subsequent analysis also excluded cells out of focus, cells whose entire feature could not be caught due to the location at the edge in the tiling processing, and cells contacted with another cell. Club cells emitting GFP-derived fluorescence were measured for cell morphology. The measurement of the cell morphology was performed with BF image data using Image J, as in Example 5 (FIG. 23B). Figure 23B indicates that the CD14⁺ cells are significantly larger in cell area than the CD14⁻ cells. These results suggest that selecting cells based on at least one of the cell markers described above (e.g., CD14+) can select cells whose area is relatively large, corresponding to Cluster 2.

### (Organoid formation from CD14* cells)

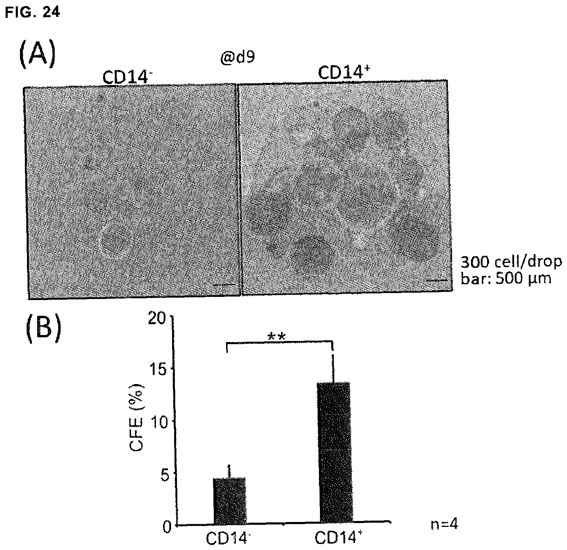

The CD14⁺ cells obtained above were examined for whether they have a high tendency to form an organoid. The CD14^{+/-} cells were seeded with a cell suspension containing the culture medium (MTEC/B27+"YHF10KNS"+2.5% Matrigel 2mL) at 300 cells per well as in Example 3 and cultured for 9 days to form organoids (FIG. 24A). Figure 24A shows that the CD14⁺ cells formed larger organoids and more in number compared to CD14⁻ cells. The percentage of cell aggregates formed by culturing the CD14^{+/-} cells (CFE [%]) also supports that the CD14⁺ cells are significantly more likely to form organoids than CD14⁻ cells (FIG. 24B).

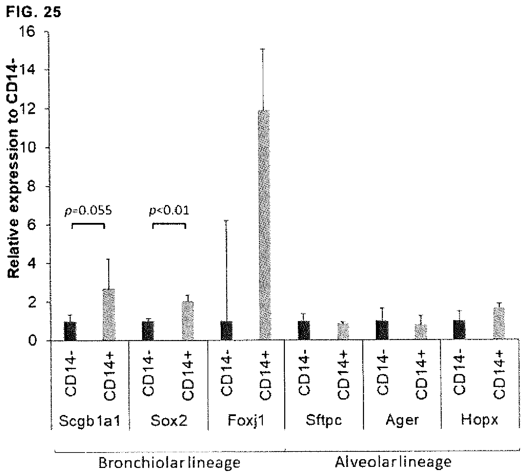

The club cells, corresponding to Cluster 2, in the organoids formed from the CD14+/- cells on day 9 after culturing were examined for the extent to which they were differentiated (FIG. 25). Figure 25 shows that both CD14⁺ and CD14⁻ cells do not differ significantly in the expression of marker genes for the Alveolar lineage, specifically Sftpc, Ager, and Hopx. On the other hand, the expression of marker genes for Bronchiolar, specifically Sox2 (bronchial epitheliumcell marker), was significantly lower in CD14⁻ cells than in CD14⁺ cells. In addition, the expressions of Scgb1a1 (club cell marker) and Foxj1 (cilia cell marker) tended to be lower in CD14⁻ cells than in CD14⁺ cells. The results indicate that the CD14⁻ cells corresponding to Cluster 0 have a higher tendency to differentiate from club cells to alveolar cells.

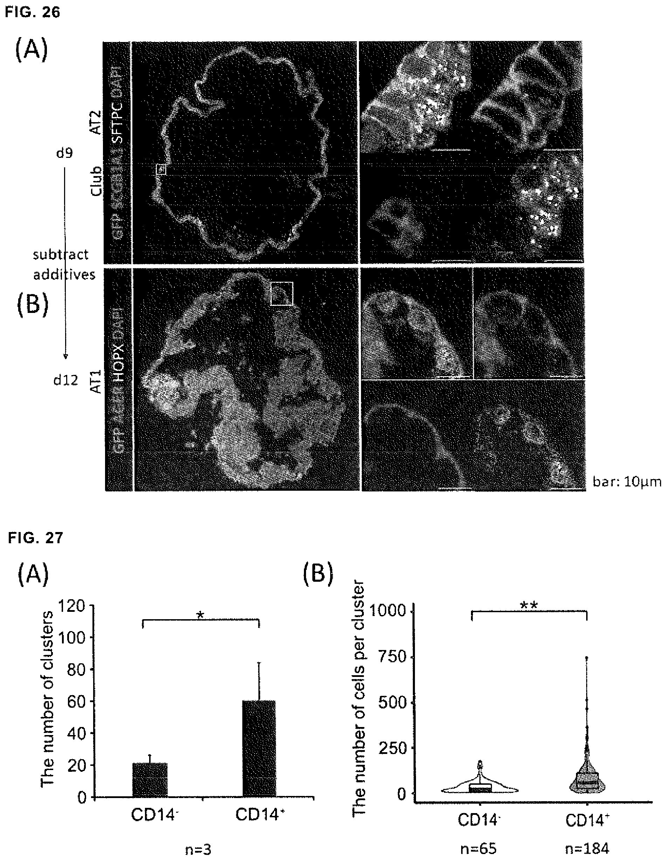

Immunostaining was performed on organoids derived from the CD14^{+/-} cells on day 9 and day 12 after culturing (on day 9 after culturing, the culture medium was exchanged with a basal medium for culturing them for 3 days to induce differentiation) (FIG. 26). Figure 26A shows that the organoid on day 9 after culturing includes cells co-expressing both a club cell marker, SCGB1A1, and an AT2 cell marker, SFTPC. Figure 26B shows the organoid on day 9 after culturing, co-expressing both AT1 cell markers, AGER and HOPX. These results indicate that CD14⁺ cells corresponding to Cluster 2 have a higher tendency to differentiate into cells of the pulmonary alveoli lineage in organoids derived from the cells described above.

### [Example 9] Transplantation of primary lung epithelial cells into a lung injury model mouse

It was examined whether the CD14^{+/-} cells can proliferate or differentiate into alveolar cells in the lungs during recovery from lung injury. CD14^{+/-} cells were collected from genetically modified mice, Scgb1a1-CreER;Rosa26-mTmG as in Example 8, and the CD14- and CD14⁺ cells were suspended in the culture medium (MTEC/B27+5% FBS+"YHF10KNS"), respectively, to prepare a CD14^{+/-} cell suspension including 7,000 cells per 75 µL of the culture medium. Bleomycin was intranasally administered at 3 mg/Kg to nude mice, BALB/cSlc-nu/nu (10 weeks old, not less than 25g) that lacked thymus and T cell function, to prepare a mouse model of lung injury before one week to inject the cell suspension. The cell suspension was injected into the lungs of the lung injury model mouse through the trachea.

The lung injury model mice were dissected to examine the survival and proliferation of the injected cells in the lungs two weeks after the injection of the cell suspension (FIG. 27). The examination used the left lungs of the mice. The left lungs were used to prepare frozen blocks, which were cut into tissue sections in a thickness of 8 µm with a microtome. Each tissue section had an interval of approximately 100 µm. GFP-derived fluorescence was checked for forty tissue sections per mouse. The viability of the cells injected into the lung was estimated by the number of clusters observed in the lung (FIG. 27A) and the number of cells included in one cluster (FIG. 27B). A population of cells emitting GFP-derived fluorescent in a distance of not more than 100 µm was defined as a cluster in Example 9

Figure 27A shows that the CD14⁺ and CD14⁻ cells were viable in the bleomycin-injured lungs, respectively. Figure 27A shows that the CD14⁺ cells were significantly more viable in the lungs than the CD14⁻ cells. Figure 27B shows that CD14⁺ cell-derived clusters were composed of significantly more cells than CD14⁻ cells-derived clusters. Fibrosis, thought to be due to inflammation, was observed in the lungs of mice injected with the CD14⁺ cells. On the other hand, the mice injected with the CD14⁻ cells showed less fibrosis in the lungs than the fibrosis in the lungs of mice injected with the CD14⁺ cells.

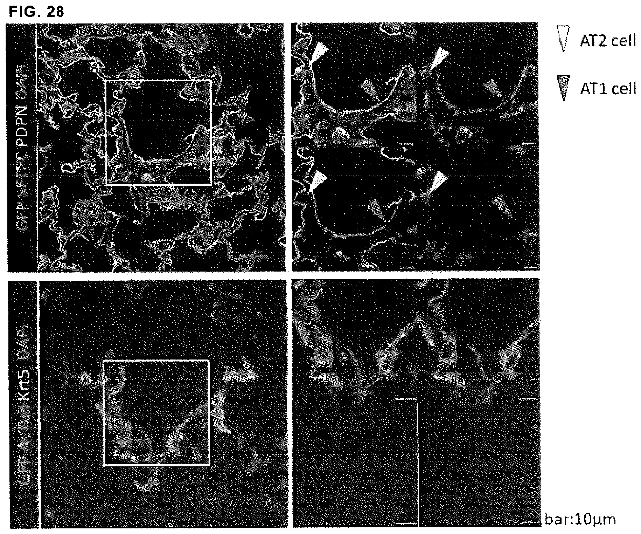

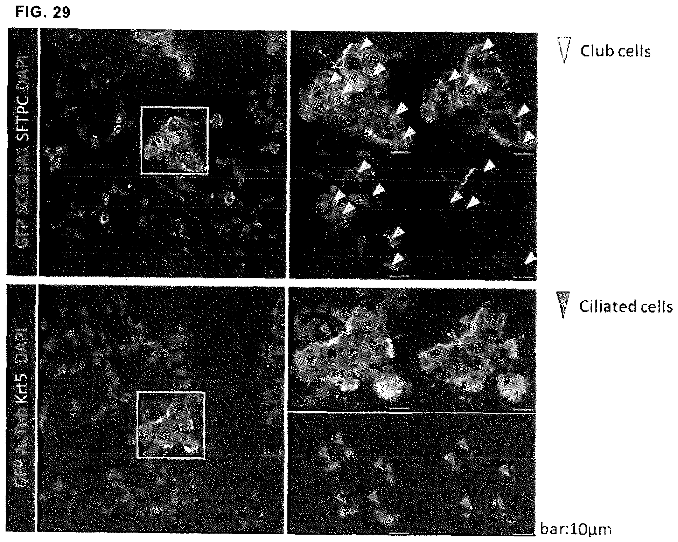

The cells injected into the lungs were examined for their differentiation capability by examining the expression of cell markers in the cells emitting GFP-derived fluorescence in the lungs. The examination used the right lungs of the mice. The right frozen lungs were used to prepare paraffin blocks, from which tissue sections having a thickness of 6 µm were prepared. Immunostaining was performed on the tissue sections. The immunostaining indicated that AT2 cells expressing GFP and an AT2 cell marker, SFTPC, and AT1 cells expressing GFP and an AT1 cell marker, PDPN, exist in the lungs injected with CD14⁺ cells. Neither ciliated cells expressing a ciliary cell marker, AcTub, nor cells expressing a basal cell marker, AcTub, were observed in the lungs. These results suggest that the injected CD14⁺ cells (GFP-positive cells) differentiated into AT2 cells and AT1 cells, which are pulmonary alveoli lineage (FIG. 28). The immunostaining further indicated that club cells expressing GFP and a club cell marker, SCGB1A1, and Ciliated cells expressing GFP and a ciliated cell marker, AcTub, exist in the lings injected with the CD14⁺ cells. The result suggests that the injected CD14⁺ cells (GFP-positive cells) differentiated into also Ciliated cells of the bronchial lineage because of the coexpression of the club cell marker SCGB1A1 and the ciliated cell marker AcTUB (FIG. 29).

Example 9 demonstrates that the cells corresponding to Cluster 0 (e.g., CD14⁻ cells) do not cause adverse effects such as inflammation in the lung to which they are delivered, possess the capability to form organoids, and can differentiate into cells of the pulmonary alveoli and bronchial lineages, suggesting that they may be useful as a regenerative medical composition for treating lung injury or lung disease. Example 9 also demonstrates that the cells corresponding to Cluster 2 (e.g., CD14⁺ cells) possess the capability to form organoids efficiently and can differentiate into cells of the pulmonary alveoli and bronchial lineages, suggesting that they may be useful as a regenerative medical composition for treating lung injury or lung disease. In addition, it suggests that the cells corresponding to Cluster 2 (e.g., CD14⁺ cells) are useful as a carrier to deliver a drug (e.g., organic compound, gene, or protein) to treat damaged lungs or lungs suffering from a disease.

## Claims

1. A method of producing an organoid derived from a lung epithelial cell or a lung cancer cell, comprising culturing a sample including the lung epithelial cell or the lung cancer cell in a culture medium, wherein
the culture medium contains 0-10% (v/v) extracellular matrix, and
a combination of at least one selected from the group consisting of keratinocyte growth factor (KGF), fibroblast growth factor (FGF) 10, and hepatocyte growth factor (HGF); bone morphogenetic protein (BMP) inhibitor; and TGFβ inhibitor, and
the culture medium is substantially free of feeder cells.

2. The method according to claim 1, wherein the combination further comprises either or both of an agent capable of activating Wnt signaling and Rho-kinase (ROCK) inhibitor.

3. The method according to claim 2, wherein the combination is
a combination of the KGF, the BMP inhibitor, and the TGFβ inhibitor;
a combination of the KGF, the agent capable of activating Wnt signaling, the BMP inhibitor, and the TGFβ inhibitor;
a combination of the FGF10, the BMP inhibitor, and the TGFβ inhibitor;
a combination of the FGF10, the agent capable of activating Wnt signaling, the BMP inhibitor, and the TGFβ inhibitor;
a combination of the HGF, the BMP inhibitor, and the TGFβ inhibitor;
a combination of the HGF, the agent capable of activating Wnt signaling, the BMP inhibitor, and the TGFβ inhibitor;
a combination of the KGF, the FGF10, the HGF, the BMP inhibitor, and the TGFβ inhibitor;
a combination of the KGF, the FGF10, the HGF, the agent capable of activating Wnt signaling, the BMP inhibitor, and the TGFβ inhibitor;
a combination of the ROCK inhibitor, the KGF, the FGF10, the HGF, the BMP inhibitor, and the TGFβ inhibitor; or
a combination of the ROCK inhibitor, the KGF, the FGF10, the HGF, the agent capable of activating Wnt signaling, the BMP inhibitor, and the TGFβ inhibitor.

4. The method according to any one of claims 1-3, wherein
the BMP inhibitor is Noggin and/or
the TGFβ inhibitor is SB431542.

5. The method according to any one of claims 2-4, wherein
the agent capable of activating Wnt signaling is CHIR99021 and/or
the ROCK inhibitor is Y-27632.

6. The method according to any one of claims 1-5, wherein the lung epithelial stem cell in the sample comprises at least one cell type selected from the group consisting of basal cell, club cell, bronchioalveolar stem cell, and alveolar epicelial cell.

7. The method according to any one of claims 1-6, comprising selecting club-I cell, AT2 cell, or club-II cell from the sample, based on at least one of cell markers corresponding to respective club-I cell, AT2 cell, and club-II cell listed in Table A and homologs thereof, before culturing the sample in the culture medium.
**Table A**
| Club-I Cell | AT2 Cell | Club-II Cell |
|---|---|---|
| Cbr2 | Lamp3 | Tff2 |
| Hp | Lyz2 | Reg3g |
| Cyp2f2 | Napsa | Bpifb1 |
| Prdx6 | Slc34a2 | Muc5b |
| Scgb1a1 | Lyz1 | Sult1d1 |
| Ldhb | Rnase4 | Fxyd3 |
| Ces1d | Hc | Scgb3a1 |
| Cckar | Cd74 | Lypd2 |
| Selenbp1 | Scd1 | Chad |
| Gstm2 | Sftpc | S100a6 |
| Scnn1b | Lgi3 | Pglyrp1 |
| Scgb1c1 | Cldn18 | Aldh3a1 |
| Plpp3 | Etv5 | Bpifa1 |
| Ephx1 | Cxcl15 | Gsto1 |
| Dcxr | S100g | Lgals3 |
| Alas1 | Elovl1 | Pigr |
| Retnla | Fas | Scgb3a2 |
| Sec14l3 | H2-Aa | Ltf |
| Ptgr1 | Fabp5 | Qsox1 |
| Krtap17-1 | Rn4.5s | Cyp2a5 |
| | | Cpd |
| | | Ly6a |
| | | Perp |
| | | Kcne3 |
| | | Il13ra1 |
| | | Slc15a2 |
| | | Cd14 |

8. An organoid derived from a lung epithelial cell or a lung cancer cell, produced by a method according to any one of claims 1-7.

9. An organoid derived from a lung epithelial cell, comprising an intracavity and a cell layer facing the intracavity, wherein
the lung epithelial cell is alveolar cell, and
the cell layer essentially consists of:
AT2 cells;
a combination of AT2 cells with cells expressing an AT2 cell marker and a bronchial epithelium marker; or
cells expressing a bronchial epithelium marker, or
the lung epithelial cell is airway cell, and
the cell layer essentially consists of a combination of basal cells with cells expressing a basal cell marker and a bronchial epithelium marker.

10. The organoid according to claim 9, wherein the lung epithelial cell is alveolar cell, the cell layer essentially consists of AT2 cells, the AT2 cells express either or both of HT2-280 and SFTPC, and the HT2-280 locally localizes in or near cell membranes of the AT2 cells, the cell membranes facing the intracavity.

11. The organoid according to claim 9, wherein
the AT2 cell marker is either or both of HT2-280 and SFTPC,
the bronchial epithelium marker is SOX2, and/or
the basal cell marker is KRT5.

12. A regenerative medical composition comprising an organoid derived from a lung epithelial cell, produced by a method according to any one of claims 1-7, an organoid derived from a lung epithelial cell according to any one of claims 9-11, and/or cells from the organoid.

13. A method of screening a substance capable of treating lung cancer, comprising
contacting a subject substance with an organoid derived from a lung cancer cell, produced by a method according to any one of claims 1-7,
measuring the organoid after contact with the subject substance, and
comparing the measured value from the organoid after contact with the subject substance to a control value.
